# EUROPÄISCHE PATENTSCHRIFT

(11) **EP 1 086 965 B1**
(45) Veröffentlichungstag und Bekanntmachung des Hinweises auf die Patenterteilung: **22.10.2003**
(21) Anmeldenummer: 00119150.1
(22) Anmeldetag: 05.09.2000
(51) Int. Cl.: C08F 220/30, C08F 251/00, C09D 133/14, C09D 151/02

(54) **Kohlenhydratlatices, Verfahren zu ihrer Herstellung und ihre Verwendung**
Carbohydrat latex, process for its prepation and its use
Latex d'hydrate de carbone, procédé de sa préparation et son utilisation

(30) Priorität: 21.09.1999 DE 19945236
(43) Veröffentlichungstag der Anmeldung: 28.03.2001
(73) Patentinhaber: Wella Aktiengesellschaft, 64274 Darmstadt (DE); Yaacoub, Emile-Joseph, Dr., 38159 Vechelde/Vallstedt (DE)
(72) Erfinder: Yaacoub, E.-J., 38159 Vechelde (DE); Koch, Ulrich, 38108 Braunschweig (DE)
(74) Vertreter: Schulze, Rainer (DE)

(56) Entgegenhaltungen:
- GB-A- 1 134 235
- GB-A- 2 091 750
- KLEIN, J. ET AL.: "Emlusion polymerization of poly(methacryloylglucose)" MACROMOL. CHEM., RAPID COMMUN. , Bd. 6, 1985, Seiten 675-678, XP001000995

## Beschreibung

Gegenstand der vorliegenden Erfindung sind Latices aus durch Emulsionspolymerisation erhaltenen Polymeren, welche aus mindestens einer Monomerart aufgebaut sind, die ausgewählt ist aus mit einer radikalisch polymerisierbaren Gruppe substituierten Kohlenhydraten oder Kohlenhydratderivaten.

Durch Emulsionspolymerisation erhältliche wässrige Polymerdispersionen (Latex) sind allgemein bekannt. Es handelt sich um fluide Systeme, die als disperse Phase Polymerteilchen und als kontinuierliche Phase Wasser oder ein wässriges Medium enthalten. Der Durchmesser der Polymerisatteilchen liegt typischerweise bei 0,01 bis 5 µm, insbesondere bei 0,01 bis 1 µm. Der Festkörperanteil kann dabei in der Regel bis zu 60% betragen, ohne daß das System hochviskos wird. Zur Stabilisierung sind in der Regel bis zu 10% Tenside und/oder Schutzkolloide enthalten. Die Tenside sind meistens anionisch, können aber auch kationisch oder nichtionisch sein. Aus J. Klein et al., *Makromol. Chem., Rapid Commun*. 6, 675-678, 1985 ist bekannt, 3-Methacryloyl-diisopropyliden-glucose in Emulsion zu polymerisieren, dann die Homopolymerisate auszufällen und chemisch zu entschützen, um wasserlösliche Polymerisate mit im Vergleich zu durch Lösungspolymerisation erhaltenen Polymeren höherer Molmasse und höherer Viskosität zu erzielen. In den Beispielen 5 und 6 GB-A-1 134 235 werden Emulsionen von bestimmten ketalisierten Saccharid/Galaktose-Acrylat-Copolymeren als Zwischenprodukte beschrieben.

Aufgabe der vorliegenden Erfindung war es, neue Polymerlatices herzustellen und der Technik neue wässrige Dispersionen in Form von dispersen Polymerpartikeln mit neuen Eigenschaften zur Verfügung zu stellen. Eine weitere Aufgabe bestand darin, hierfür Polymere zu verwenden, die zumindest teilweise auf natürlichen, nachwachsenden Rohstoffen basieren.

Gegenstand der vorliegenden Anmeldung ist der Polymerlatex gemäß Patentanspruch 1, enthaltend mindestens ein in Wasser dispergiertes, durch Emulsionspolymerisation hergestelltes Polymer, dessen in disperser Verteilung befindliches Polymerisat in radikalisch polymerisierter Form aufgebaut ist aus
(A)mindestens einer ersten Monomerart, welche eine mit mindestens einer radikalisch polymerisierbaren Gruppe substituiertes Kohlenhydrat oder Kohlenhydratderivat ist, welche vorzugsweise ausgewählt ist aus Sacchariden, Saccharidderivaten und Zuckeralkoholen, wobei die Monomerart (A) eine oder mehrere Schutzgruppen enthalten kann und
(B)mindestens einer zweiten, von (A) verschiedenen, mit (A) radikalisch copolymerisierbaren Monomerart.

Die erfindungsgemäßen Polymerlatices sind dadurch charakterisiert, daß die Kohlenhydratkomponenten ihnen ihre spezifischen Qualitäten aufprägen. Zu diesen zählen Hydrophilie bzw. eine charakteristische Balance zwischen Hydrophilie und Hydrophobie, Kompatibilität insbesondere mit biologischen Systemen, und hier z.B. speziell mit der Haut, keine oder geringe Toxizität, biologische Abbaubarkeit usw..

In einigen Fällen kann es wegen der Eigenschaften der entstehenden Polymerlatices interessant sein,
- zwei der unter (A) angegebenen Monomerarten oder
- zwei Zuckermonomere (A) und ein Monomer der unter (B) angegebenen Verbindungen oder
- ein Zuckermonomer (A) und zwei bzw. drei Monomere (B) einzusetzen.

Die Monomerart (A) ist vorzugsweise nur mit einer einzigen radikalisch polymerisierbaren Gruppe substituiert. Bei Verwendung von mit zwei oder mehr radikalisch polymerisierbaren Gruppen substituierten Monomeren können vernetzte Polymerisate erhalten werden.

Erfindungsgemäße, die Monomerart (A) enthaltende Copolymere weisen die allgemeine Formel (I) auf

-[CH₂-C(Y) (D-A)]ₘ-[B]ₙ- (I)

wobei
- Y: für R oder COOR und R für H oder für einen C1- bis C18-Kohlenwasserstoffrest steht,
- D: für Sauerstoff, CONH oder vorzugsweise COO steht,
- A: für ein Saccharid, eine Saccharidderivat oder für einen Zuckeralkohol steht und eine oder mehrere Schutzgruppen enthalten kann,
- B: für ein radikalisch polymerisierbares Monomer steht, welches keine Gruppe A enthält und
- m und n: für Zahlen größer Null stehen, die den jeweiligen Monomergehalt angeben.

Der Rest Y ist vorzugsweise ausgewählt aus Wasserstoff, Methyl, -COOH, -COOCH₃ und -COOC₂H₅. Die Zahlen m und n sind vorzugsweise so gewählt, daß sich für das Gesamtpolymer ein mittleres Molekulargewicht von 10.000 bis 5.000.000 ergibt.

Monomerart (A) und Comonomer (B) liegen vorzugsweise in einem Verhältnis von 2:98 bis 98:2, besonders bevorzugt von 20:80 bis 80:20, ganz besonders bevorzugt von 30:70 bis 70:30 vor.

Die Monomerart (A) bzw. der Rest A in Formel (I) kann von einem Mono-, Di-, Oligo- oder Polysaccharid abgeleitet sein, ist aber vorzugsweise von einem Monosaccharid oder einem Zuckeralkohol oder deren Derivaten abgeleitet. Derivate sind chemisch geschützte oder enzymatisch oder chemisch modifizierte Verbindungen. Geeignete Derivate sind beispielsweise solche, die an den OH-Gruppen alkyliert, acyliert oder mit Schutzgruppen versehen sind. Besonders bevorzugt sind Monosaccharide oder Zuckeralkohole mit fünf Hydroxylgruppen, die eine solche Konfiguration aufweisen, daß zweimal zwei Hydroxylgruppen durch je eine Alkylidengruppe geschützt werden können und die fünfte Hydroxylgruppe mit der radikalisch polymerisierbaren Gruppe substituiert ist. Besonders bevorzugt sind Derivate von Glucose, Fructose, Galactose, Sorbose oder Xylit, welche eine oder mehrere, vorzugsweise zwei zweiwertige Schutzgruppen enthalten können. Bevorzugte Schutzgruppen sind Cyclohexyliden und insbesondere Isopropyliden. Es können jeweils sowohl die D- als auch die L-Enantiomeren oder ein D,L-Enantiomerengemisch eingesetzt werden.

Vorzugsweise weist die Monomerart (A) die allgemeine Formel (III) auf 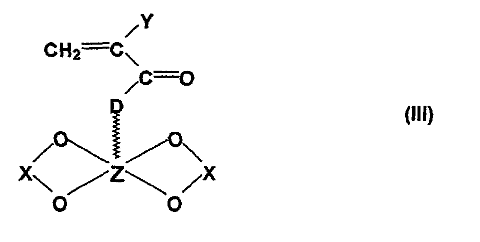 wobei Z für einen von einem Saccharid, einem Saccharidderivat oder einem Zuckeralkohol abgeleiteten Rest steht, insbesondere für ein Monosaccharid, ein Monosaccharidderivat oder einen Zuckeralkohol, vorzugsweise einen Glucose- oder Fructoserest, D für Stickstoff oder vorzugsweise für Sauerstoff steht, X eine chemische Schutzgruppe, vorzugsweise eine Isopropyliden- oder eine Cyclohexylidengruppe und Y einen der Reste R oder -COOR bedeuten und R Waserstoff oder einen C₁- bis C₁₈-Alkylrest bedeutet. Vorzugsweise ist Y Wasserstoff oder Methyl.

Beispiele für als Monomerart (A) geeignete Saccharidacrylate sind
- 3-O-(Meth)acryloyl-1,2:5,6-di-O-isopropyliden-α-D-glucofuranose (3-MDG bzw. 3-ADG) und seine Derivate wie z.B. 3-O-(Meth)acryloyl-1,2:5,6-di-O-cyclohexyliden-α-D-glucofuranose, wenn als Rest Z D-Glucose das Ausgangsmaterial darstellt,
- 3-O-(Meth)acryloyl-1,2:4,5-di-O-isopropyliden-α-D-fructopyranose (3-MDF bzw. 3-ADF) und seine Derivate wie z.B. 3-O-(Meth)acryloyl-1,2:4,5-di-O-cyclohexyliden-α-D-fructopyranose, wenn als Rest Z D-Fructose das Ausgangsmaterial darstellt,
- 1-O-(Meth)acryloyl-2,3:4,5-di-O-isopropyliden-α-D-fructopyranose (1-MDF bzw. 1-ADF) und seine Derivate wie z.B. 1-O-(Meth)acryloyl-2,3:4,5-di-O-cyclohexyliden-α-D-fructopyranose, wenn als Rest Z D-Fructose das Ausgangsmaterial darstellt,
- 1-O-(Meth)acryloyl-2,3:4,6-di-O-isopropyliden-α-D-sorbofuranose (1-MDS bzw. 1-ADS) und seine Derivate wie z.B. 1-O-(Meth)acryloyl-2,3:4,6-di-O-cyclohexyliden-α-D-sorbofuranose, wenn als Rest Z D-Sorbose das Ausgangsmaterial darstellt,
- 6-O-(Meth)acryloyl-1,2:3,4-di-O-isopropyliden-α-D-galactopyranose (6-MDGa bzw. 6-ADGa) und seine Derivate wie z.B. 6-O-(Meth)acryloyl-1,2:3,4-di-O-cyclohexyliden-α-D-galactopyranose, wenn als Rest Z D-Galactose das Ausgangsmaterial darstellt,
- 5-O-(Meth)acryloyl-1,2:3,4-di-O-isopropyliden-α-DL-xylit (5-MDXy bzw. 5-ADXy) und seine Derivate wie z.B. 5-O-(Meth)acryloyl-1,2:3,4-di-O-cyclohexyliden-α-DL-xylit, wenn als Rest Z ein Xylit das Ausgangsmaterial darstellt,
- 3-O-(Meth)acryloyl-1,2:4,5-di-O-isopropyliden-xylitol (3-MDXy bzw. 3-ADXy) und seine Derivate wie z.B. 3-O-(Meth)acryloyl-1,2:4,5-di-O-cyclohexyliden-xylitol, wenn als Rest Z ein Xylit das Ausgangsmaterial darstellt.

Das Comonomer B ist vorzugsweise ausgewählt aus den folgenden Gruppen, wobei auch Mischungen der genannten Monomer eingesetzt werden können:
(a) monoethylenisch ungesättigte C3- bis C12-Carbonsäuren oder deren Salze,
(b) monoethylenisch ungesättigte Ester von C3- bis C12-Carbonsäuren und C1- bis C14-Alkoholen,
(c) Acrylsäure- oder Methacrylsäuredialkylaminoalkylester mit insgesamt bis zu 30 C-Atomen im Dialkylaminoalkyl-Rest, welche in N-quaternisierter Form oder in Salzform vorliegen können,
(d) Amide der unter (a) genannten ungesättigten Carbonsäuren, z.B. Acrylsäureamid, Methacrylsäureamid, N,N-Dialkyl-acrylsäure- oder -methacrylsäureamid,
(e) fünf- bis achtgliedrige N-Vinyllactame, welche am Ring durch bis zu drei C1- bis C12-Alkylreste substituiert sein können,
(f) Maleinsäure-, Fumarsäure- und Itakonsäuredialkylester mit bis zu 12 C-Atomen pro Alkylrest,
(g) monoethylenisch ungesättigte C3- bis C12-Alkylvinylether,
(h) Vinylaromaten, wie z.B. Styrol und Styrolderivate, welche am aromatischen Ring durch ein bis drei C1-bis C12-Alkylreste substituiert sein können,
(i) ein- oder mehrfach ungesättigte Kohlenwasserstoffe, wie z.B. Ethylen, Propylen oder 1,3-Butadien,
(j) Vinylhalogenide, wie z.B. Vinylchlorid.

Geeignete Comonomere (B) sind beispielweise:
(a) Acrylsäure, Methacrylsäure, Dimethylacrylsäure, Ethylacrylsäure, Vinylessigsäure, Allylessigsäure und Vinylpropionsäure, Fumarsäure, Maleinsäure, Itakonsäure. Vorzugsweise verwendet man aus dieser Gruppe Acrylsäure, Methacrylsäure, Itakonsäure deren Gemische sowie die Natrium-, Kalium-, Calcium- oder Ammoniumsalze oder deren Mischungen.
(b) in dieser Gruppe finden sich z.B. Alkyl-, Hydroxyalkyl- und Vinylester wie Methacrylat, Ethylacrylat, n-Propylacrylat, Isopropylacrylat, n-Butylacrylat, tert.-Butylacrylat, 2-Ethylhexylacrylat, Methylmethacrylat, Ethylmethacrylat, Isopropylmethacrylat, n-Butylmethacrylat, Isobutylmethacrylat, Hydroxyethylacrylat, Hydroxypropylacrylat, Hydroxybutylacrylat, Hydroxyethylmethacrylat, 2-Ethylhexylmethacrylat und Vinylformiat, Vinylacetat, Isopropenylacetat, Vinylpropionat, Vinyl-2-ethylhexanoat, Vinyllaurat sowie Mischungen derselben Verbindung.
(c) in dieser Gruppe kommen z.B. in Betracht: Dimethylaminoethylacrylat, Diethylaminoethylacrylat, Methylethylaminoethylacrylat, Di-tert.-butylaminoethylacrylat, Dimethylamino-methyl(oder butyl, hexyl, octyl, stearyl)-acrylat, Dimethyl-(oder Diethyl, Methylethyl, Di-tert.-butyl)-aminoethylmethacrylat, Dimethylaminomethyl-(oder butyl, amyl, hexyl, octyl, stearyl)-methacrylat.
(d) Acrylsäureamid, Methacrylsäureamid, N,N-Dimethylacrylsäureamid, N,N-Dimethyl-methacrylsäureamid.
(e) Als N-Vinyllactame sind beispielsweise 1-Vinylpyrrolidon, 1-Vinylcaprolactam, 1-Vinylpiperidon, 4-Methyl-1-vinylpyrrolidon, 3,5-Dimethyl-1-vinylcaprolactam geeignet.
(f) Fumarsäurediethylester, Fumarsäuredimethylester, Fumarsäuredicyclohexylester, Maleinsäurediethylester, Maleinsäuredimethylester, Itakonsäuredimethylester, Itakonsäurediethylester.
(g) in dieser Gruppe kommen z.B. in Betracht: Alkyl- und Hydroxyalkylvinylether wie Methyl-, Ethyl-, Propyl-, Isopropyl-, Butylvinylether, Hydroxyethylvinylether, Hydroxypropylvinylether, Hydroxybutylvinylether.

Ein weiterer Gegenstand der Erfindung ist ein Polymerlatex, enthaltend in Wasser dispergierte Komposit-Polymerpartikel, insbesondere solche mit einer Kern/Schale-Morphologie. Die Komposit-Polymerpartikel sind durch mindestens zweistufige Emulsionspolymerisation hergestellt und aufgebaut aus einem oder mehreren Polymeren, wobei mindestens ein Polymer in radikalisch polymerisierter Form aufgebaut ist aus mindestens einer der oben näher beschriebenen Monomerart (A). Die Komposit-Polymerpartikel weisen vorzugsweise eine Kern/Schale-Morphologie auf, aber auch von dieser Idealform abweichende Morphologien sind möglich.

Bei den erfindungsgemäßen Komposit-Polymerlatices kann das die Monomerart (A) enthaltende Polymer ein Homopolymer sein oder es kann ein durch Copolymerisation mit mindestens einem Monomer der oben näher beschriebenen Monomerart (B) erhältliches Polymer sein. Es kann sowohl im Polymerisat der ersten Stufe (Kern) als auch im Polymerisat der zweiten Stufe (Schale) enthalten sein oder es ist nur im Polymerisat der ersten Stufe (Kern) enthalten und das Polymerisat der zweiten Stufe (Schale) ist aus einem oder mehreren, beliebigen anderen Polymeren aufgebaut, welche keine Monomerart (A) enthalten. Bevorzugt sind jedoch Komposit-Polymerpartikel, bei denen das Polymerisat der ersten Stufe (Kern) aus einem oder mehreren Polymeren aufgebaut ist, welche keine Monomerart (A) enthalten und das Polymerisat der zweiten Stufe (Schale) aus mindestens einem Homo- oder Copolymer gebildet wird, welches mindestens eine Monomerart (A) enthält.

Für den Aufbau der Komposit-Polymerpartikel geeignete Homopolymere aus der Monomerart (A) weisen vorzugsweise die allgemeine Formel (II) auf

-[CH₂-C(Y) (D-A)]ₘ- (II)

wobei
- Y: für R oder COOR und R für H oder für einen C1- bis C18-Kohlenwasserstoffrest steht,
- D: für Sauerstoff, CONH oder vorzugsweise COO steht,
- A: für ein Saccharid, ein Saccharidderivat oder für einen Zuckeralkohol steht und eine oder mehrere Schutzgruppen enthalten kann und
- m: für eine Zahl größer Null steht, die den Polymerisationsgrad angibt und vorzugsweise so gewählt ist, daß sich für das Gesamtpolymer ein mittleres Molekulargewicht von 10.000 bis 5.000.000 ergibt.

Für den Substituenten A sind die gleichen Gruppen geeignet und bevorzugt wie oben bei Formel (I) beschrieben. Vorzugsweise steht Y für Wasserstoff oder Methyl, A für ein Monosaccharid, ein Monosaccharidderivat oder einen Zuckeralkohol, insbesondere für Glucose, Fructose, Galactose, Sorbose oder Xylit. Bei den Schutzgruppen, von denen vorzugsweise zwei pro Zuckermolekül vorhanden sind, handelt es sich vorzugsweise um Isopropyliden oder um Cyclohexyliden.

Der Durchmesser der Komposit-Polymerpartikel bzw. der Kern/Schale-Polymerisatteilchen beträgt vorzugsweise 20 bis 3000 nm und der Gewichtsanteil des Polymerisats der zweiten Stufe (Schale) beträgt vorzugsweise 2 bis 90%, besonders bevorzugt 5 bis 50%, ganz besonders bevorzugt 10 bis 30% des Gesamtgewichts der Partikel.

Ein weiterer Gegenstand der vorliegenden Erfindung ist ein Verfahren zur Herstellung der vorstehend definierten saccharidhaltigen Polymerlatices. Hierbei werden die Monomere zur Bildung des dispers verteilten Polymerisats mittels Emulsionspolymerisation radikalisch polymerisiert, d.h. das Polymerisat wird aus den wenigstens eine ethylenisch ungesättigte Gruppe aufweisenden Monomeren im Beisein von Emulgatoren und gegebenenfalls weiteren, beispielsweise polymeren Dispergiermitteln sowie radikalischen Polymerisationsinitiatoren unmittelbar in wässrigem Medium in disperser Verteilung befindlich erzeugt.

Hierbei kann das Verfahren so gesteuert werden, daß Saccharidlatices mit maßgeschneiderten Eigenschaften hergestellt werden:
- Die gewünschte Endzusammensetzung der Saccharidlatices kann in Hinblick auf die gewünschten Eigenschaften und industriellen Anwendungen gezielt beim Herstellungsverfahren eingestellt werden. Beispielsweise kann durch Verwendung von verschiedenen Mengen oder verschiedenartigen hydrophoben Comonomeren wie z.B. Acrylatestern, Styrol oder Butadien die Hydrophilie/Hydrophobie der Latices eingestellt werden.
- Je nach gewünschen Eigenschaften können Saccharidlatices mit relativ niedrigem Molekulargewicht (kleiner als etwa 20.000), mittlerem Molekulargewicht (etwa 20.000 bis 100.000) oder mit hohem Molekulargewicht (über 100.000) hergestellt werden
- Durch Wahl der Monomere und Comonomere können die mit der Monomerzusammensetzung und Morphologie eng zusammenhängenden thermische Eigenschaften der erfindungsgemäßen Saccharidlatices definiert eingestellt werden.
- Die gewünschte Verträglichkeit (Löslichkeit) oder Verdünnbarkeit solcher wässrigen Dispersionen mit niedermolekularen Verbindungen wie z. B. Alkoholen, insbesondere Methanol, Ethanol oder Isopropanol, bei der Formulierung kann erreicht werden, indem man einerseits verschiedene ionische und/oder nichtionische Emulgatoren oder andererseits neben den Zuckeracrylatmonomeren ein, zwei oder drei weitere Comonomere mit verschiedener Hydrophilie oder Hydrophobie einsetzt.
- Bei vielen Emulsionen sind die rheologischen Eigenschaften von großer Bedeutung. Viskosität und Streichfähigkeit sind entscheidende Qualitätsmerkmale. Diese können im wesentlichen durch die Einstellung der Partikelgröße sowie der Partikelgrößenverteilung beeinflußt werden.
- Auch weitere Qualitätsmerkmale der Emulsionen wie die optischen Eigenschaften (z.B. Farbe, Glanz), die Wirkstofffreisetzung sowie die Verteilung von Inhaltstoffen lassen sich durch geeignete Wahl der Partikelgröße gestalten oder beeinflussen.

Die Emulsionspolymerisation kann nach bekannten Verfahren zur Polymerisation in wässriger Emulsion erfolgen, wie z.B.
a) in einem Batch-Verfahren, bei dem die gesamte Monomerzugabe in einer einzelnen Charge erfolgt,
b) in einem halb-kontinuierlichen Verfahren, z.B. dem sogenannten Prä-Emulsionverfahren, bei dem zunächst nur ein Teil der Monomeren zugegeben und polymerisiert wird und die weitere Monomerzugabe inkrementell erfolgt oder
c) in einem kontinuierlichem Verfahren, bei dem die Monomerzugabe kontinuierlich über einen längeren Zeitraum hinweg erfolgt oder
d) in einem Verfahren, welches zweistufig ist, wobei in der ersten Stufe eine Dispersion eines Saat-Polymerisats gebildet oder vorgelegt wird und in einer zweiten Stufe hieraus durch Emulsionspolymerisation ein Komposit-Polymerlatex gebildet wird oder
e) in einem Verfahren, welches als sogenanntes Shot-Growth-Verfahren ausgestaltet ist, wobei zunächst eine erste Monomerart oder ein erstes Monomergemisch teilweise polymerisiert wird und anschließend, zu einem Zeitpunkt, zu dem das erste Monomergemisch oder die erste Monomerart noch nicht vollständig, z.B. erst zu 80-90% umgesetzt ist, eine zweite Monomerart oder ein zweites Monomergemisch zugegeben und polymerisiert wird.

Die gesamte Menge des wässrigen Mediums mit den Polymerisationszusätzen kann vor Einführung der Monomeren im Polymerisationsgefäß vorliegen, oder das wässrige Medium oder ein Teil hiervon kann alternativ kontinuierlich oder inkrementell im Verlauf der Polymerisation zugegeben werden. Es können übliche Saat-Verfahren zur Unterstützung der Steuerung der Polymerisation eingesetzt werden, um die gewünschte durchschnittliche Teilchengröße und Teilchengrößenverteilung zu erreichen. Falls ein Saat-Verfahren eingesetzt wird, liegt die Polymersaat typischerweise in einer Menge vor, die etwa 0,1 bis 60 Gew.% des gesamten Polymers entspricht, und die Größe der Saatpartikel reicht typischerweise von etwa 10% bis 90% des Durchmessers der herzustellenden Polymerteilchen. Der Saatlatex kann aus einem zuvor hergestellten Latex oder aus einem dispergierten Polymerpulver bestehen, oder er kann in situ hergestellt werden. Die Monomerzusammensetzung des Saatlatex kann variieren, es ist jedoch bevorzugt, daß sie im wesentlichen der des Polymers entspricht.

Die bei der Herstellung der Latex-Polymere einzusetzenden Monomere sowie gegebenenfalls die Comonomere und wahlweise die Saat werden unter ausreichender Bewegung in Wasser dispergiert, um das Gemisch zu emulgieren. Das wässrige Medium kann auch einen radikalischen Initiator, ein Emulgiermittel (Tensid) oder andere Bestandteile enthalten, die bekannt sind und üblicherweise in der Technik als Hilfsstoffe für Emulsionspolymerisationen eingesetzt werden. Die Polymerisation wird durch Radikalbildner in einem Temperaturbereich von 0 bis 90°C initiiert, vorzugsweise unter Erwärmen des emulgierten Gemisches unter ständiger Bewegung auf eine Temperatur von üblicherweise zwischen etwa 20 und 90°C, vorzugsweise zwischen 40 und 80°C, besonders bevorzugt zwischen 50 und 80°C. Die Polymerisation wird fortgeführt, indem das emulgierte Gemisch auf der gewählten Temperatur gehalten wird, bis der gewünschte Umsetzungsgrad für die Umwandlung des Monomers oder der Monomere zum Polymer erreicht worden ist. Nach Beendigung der Monomerzugabe kann solange Initiator nachdosiert werden, bis der Restmonomergehalt bezogen auf das Gesamtgewicht des Latex unter 1 Gew.% liegt. Vorzugsweise wird die Polymerisation des Latex so geführt, daß der Restmonomergehalt unter 1 Gew.%, bezogen auf das Gesamtgewicht des Latex beträgt und ein Feststoffgehalt von ca. 20 bis 65 Gew.% resultiert.

Bezogen auf den bei der Polymerisation eingesetzten Gesamtgehalt an Monomeren verwendet man vorzugsweise 0,01 bis 20, besonders bevorzugt 0,1 bis 10 Mol.-% eines geeigneten radikalischen Polymerisationsinitiators oder einer Mischung mehrerer Polymerisationsinitiatoren. Man kann wasserlösliche sowie wasserunlösliche Initiatoren oder Mischungen von wasserlöslichen und wasserunlöslichen Initiatoren einsetzen. Die in Wasser unlöslichen Initiatoren sind dann in der dispergierten organischen Phase (Ölphase) löslich. Als Initiatoren kommen zur Herstellung der erfindungsgemäßen Polymerdispersion prinzipiell alle diejenigen in Betracht, die in der Lage sind, eine radikalische, wässrige Emulsionspolymerisation auszulösen. Es kann sich dabei sowohl um Peroxide als auch um Persulfate, Peroxodisulfate oder um Azoverbindungen handeln. Besonders geeignete Peroxide sind Wasserstoffperoxid, tert.-Butylperoxid, Diisopropylbenzolhydroperoxid, Para-Menthanhydroperoxid, Cumolhydroperoxid und Peroxodischwefelsäure sowie deren Salze. Geeignete Azoverbindugnen sind Azobisisobutyronitril oder Azobiscyanovaleriansäure.

Falls man die Polymerisation zunächst bei niedriger Temperatur startet und bei höherer Temperatur zu Ende führt, ist es zweckmäßig, mit mindestens zwei bei verschiedenen Temperaturen zerfallenden Initiatoren zu arbeiten, nämlich zunächst mit einem bei niedriger Temperatur zerfallenden Initiator zu beginnen und dann die Hauptpolymerisation mit einem Initiator zu Ende zu führen, der bei höherer Temperatur zerfällt. Für die folgenden angegebenen Temperaturbereiche kann man beispielsweise die dafür aufgeführten Initiatoren verwenden:
Temperatur 40 bis 60°C:
   Acetylcyclohexansulfonylperoxid, Diacetyl-, Dicyclohexyl- oder Di-2-ethylhexylperoxidicarbonat, tert.-Butyl- oder tert.-Amylperneodecanoat, 2,2'-Azobis-(4-methoxy-2,4-dimethyl-valeronitril), 2,2'-Azobis-(2-amidinopropan)-dihydrochlorid, 2,2'-Azobis-(2-(2-imidazolin-2-yl)-propan)-dihydrochlorid;
Temperatur 60 bis 80°C:
   Natriumpersulfat, Kaliumpersulfat, Ammoniumpersulfat, Tert.-Butyl-, tert.-Amylperpivalat, Dioctanoyl- oder Dilaurylperoxid, 2,2'-Azobis-(2,4-dimethylvaleronitril), 2,2'-Azobis-(isobutyronitril);
Temperatur 80 bis 90°C:
   Dibenzoylperoxid, tert.-Butylper-2-ethylhexanoat, tert.-Butylpermaleinat, Dimethyl-2,2'-azobis-isobutyrat.

Wenn man die Halbwertszeiten der angegebenen radikalbildenden Initiatoren verringern will, verwendet man zusätzlich zu den genannten Initiatoren noch als Redoxkatalysatoren geeignete Salze, d.h. kombinierte Systeme, die aus wenigstens einem Reduktionsmittel und wenigstens einem Oxidationsmittel zusammengesetzt sind. Geeignete Oxidationsmittel sind beispielsweise Peroxide oder Hydroperoxide wie z.B. Benzoylperoxid oder Wasserstoffperoxid oder Kalium-, Natrium- oder Ammoniumpersulfat. Die Reduktionsmittel aktivieren die Radikalbildung und ermöglichen so die Durchführung der Emulsionspolymerisation bei tieferen Temperaturen, beispielsweise bei 20 bis 55°C. Als Reduktionsmittel setzt man bezogen auf den Redoxinitiator 0,01 bis 50 Mol.-% der reduzierend wirkenden Verbindungen ein. Die reduzierende Komponente von Redoxkatalysatoren kann beispielsweise von Verbindungen wie Natriumsulfit, Natriumbisulfit, Alkalithiosulfat, Natriumformaldehydsulfoxylat, Mercaptane oder Hydrazin gebildet werden. Weitere geeignete Reduktionsmittel sind Ascorbinsäure, Acetonbisulfit, das Natriumsalz der Hydroxymethansulfinsäure oder Natriumdithionit. Bevorzugt sind auch kombinierte Systeme mit einem zusätzlichem Gehalt an einer geringen Menge einer löslichen Metallverbindung, welche in mehreren Wertigkeitsstufen auftreten kann, beispielsweise Eisen(II)salze wie Eisen(II)sulfat. Anstelle eines wasserlöslichen Eisen(II)salzes wird häufig auch eine Kombination von Fe/V-Salzen benutzt. Der Vorteil dieser Systeme liegt darin, daß sie eine Durchführung der Emulsionpolymerisation bei noch niedrigeren Temperaturen ermöglichen. Beispiele für derartige Systeme sind Ascorbinsäure/Eisen(II)sulfat/ Wasserstoffperoxid oder Natriumdithionit/Natriumformaldehydsulfoxylat/Eisen(II)sulfat/ Para-Menthanhydroperoxid oder Diisopropylbenzolhydroperoxid. Häufig wird derartigen Systemen noch ein Chelatbildner zugesetzt, um sicherzustellen, daß sich die metalische Komponente in Lösung befindet und dem Reaktionssystem nicht durch Ausfällung entzogen wird. Ein solcher Chelatbildner ist z.B. das Natriumsalz der Ethylendiamintetraessigsäure. Häufig wird die metallische Komponente unmitelbar als Chelatkomplex zugesetzt.

Zur Erhöhung der Stabilität der erfindungsgemäß hergestellten Polymerdispersion, insbesondere bei höheren Feststoffgehalten, können sowohl die zur Durchführung von radikalischen, wässrigen Emulsionspolymerisationen üblicherweise eingesetzten Schutzkolloide als auch Emulgatoren oder deren Mischungen oder Elektrolyte wie z.B. NaCl eingesetzt werden. Die vorzugsweise wasserlöslichen Stabilisatoren können bezogen auf den Monomergehalt in Mengen von 1 bis 20 Gew.% zugesetzt werden. Geeignete Schutzkolloide sind beispielsweise Polyvinylalkohole, Cellulosederivate, Polyvinylpyrrolidon oder Polykondensate aus Naphtalinsulfonsäure und Formaldehyd mit einem mittleren relativen Molekulargewicht von 4000 bis 8000 oder amphiphile Blockcopolymere, die aus einem hydrophoben und aus einem hydrophilen Segment bestehen, mit einem Molekulargewicht von 5000 bis 80 000.

Als geeignete Emulgatoren, die im Unterschied zu den Schutzkolloiden als grenzflächenaktive Substanzen Micellen bilden können, werden bevorzugt solche eingesetzt, deren Molekulargewichte im Gegensatz zu den Schutzkolloiden üblicherweise unter 2000, vorzugsweise unter 1000 liegen. Die Tenside werden in einer Menge von 1 bis 20 Gew.% bezogen auf den Monomergehalt eingesetzt. Die Emulgatoren können nichtionisch, anionisch oder kationisch sein. Mischungen können eingesetzt werden, soweit sie miteinander verträglich sind, was in der regel der Fall ist mit Ausnahme von Mischungen von kationischen und anionischen Emulgatoren, die häufig miteinander unverträglich sind. Üblicherweise wird mindestens ein anionischer Emulgator verwendet. Ein oder mehrere nichtionische Emulgatoren können zusätzlich verwendet werden. Geeignete anionische Emulgatoren sind z.B. Alkali- und Ammoniumsalze von Alkylsulfaten, insbesondere von C8- bis C16-Alkylsulfaten, von Aryl- und Alkylarylsulfonaten, insbesondere C9- bis C18-Alkylarylsulfonaten, von Schwefelsäurehalbestern ethoxylierter Alkanole und Alkylphenole, insbesondere von C12- bis C18-Alkanolen mit einem Ethoxylierungsgrad von 1 bis 70, von C12- bis C18-Alkylsulfonsäuren, von Arylsulfonsäuren, von C9- bis C18-Alkylarylsulfonsäuren und von C10- bis C18-Alkylsulfaten, sowie sulfonierte Alkylester und Fettsäureseifen. Geeignete nichtionische Emulgatoren sind z.B. ethoxylierte Mono-, Di- und Tri- C4- bis C12-alkylphenole mit einem Ethoxylierungsgrad von 3 bis 100 oder ethoxylierte C8- bis C18-Fettalkohole mit eidnem Ethoxylierungsgrad von 3 bis 100. Weiterhin sind Sulfobernsteinsäureester geeignet. Spezielle Beispiele geeigenter Emulgatoren sind u.a. Natriumlaurylsulfat, Natriumdodecylbenzolsulfonat, Natriumbutylnaphthalinsulfonat, Dinatriumdodecyldiphenylethersulfonat, N-Octadecyldinatriumsulfosuccinat und Dioctylnatriumsulfosuccinat. Die Emulgiermittel werden in geeigneten Mengen eingesetzt, um eine angemessene Emulgierung zu erreichen und die gewünschte Teilchengröße und Teilchengrößenverteilung zu liefern.

Weiterhin können bezogen auf den Monomergehalt 0,1 bis 10 Gew.%, vorzugsweise 0,5 bis 5 Gew.% an multifunktionellen Verbindungen, insbesondere mindestens zweifach ethylenisch ungesättigten, quervernetzenden Verbindungen oder Pfropfungsmitteln zugesetzt werden. Geeignete Vernetzer sind z.B. Divinylbenzol, Diacrylate von Polyethylenglykol wie z.B. Ethylenglykoldimethacrylate, Diacrylate von Polypropylenglykol, N,N'-bis-methylenacrylamid, Divinylether der aliphatischen Diole, Diallylether, 1,7-Octadien, 1,9-Decadien, Triallylamin, Diallylphtalat oder Tetraallylethylendiamin. Geeignete Pfropfungsmittel sind beispielsweise Acryl- oder Methacrylsäureallylester, Acryl- oder Methacrylsäuremethallylester, Fumar-, Malein- oder Itaconsäuremono- oder -diallylester oder Fumar-, Malein- oder Itaconsäuremono- oder -dimethallylester.

Zur Begrenzung des Vernetzungs- und/oder Polymerisatonsgrades können der zu polymerisierenden Mischung bezogen auf den Monomergehalt 0,01 bis 10, bevorzugt 0,1 bis 3 Mol% Regler, sogenannte Molekulargewichtsregler (Kettenüberträger) zugesetzt werden. Geeignete Regler sind Mercaptane, insbesondere C3- bis C15-Alkanthiole, sowie Aldehyde und Chlorkohlenwasserstoffe. Bevorzugt sind tert.-Docedylmercaptan und n-Dodecylmercaptan.

Die Emulsionspolymerisation erfolgt üblicherweise in einer Inertgasatmosphäre unter Ausschluß von Luftsauerstoff. Das Wasser wird zweckmäßig unmittelbar vor dem Polymerisationsansatz frisch destilliert und entgast. Die Monomerarten (A) und (B) sind vorzugsweise rein und stabilisatorfrei und liegen in gelöster Form vor. Während der Polymerisation wird für eine gute Durchmischung der Reaktionsteilnehmer gesorgt. Die Herstellung der erfindungsgemäßen Saccharidlatices kann in üblichen Polymerisationsvorrichtungen durchgeführt werden. Hierzu verwendet man beispielsweise Rührkessel oder Doppelwandreaktoren, die mit einem Anker-, Blatt-, Impeller- oder einem mehrstufigen Impulsgegenstromrührer ausgestattet sind.

Die radikalische, wässrige Emulsionspolymerisation zur Erzeugung der erfindungsgemäßen Polymerlatices wird vorteilhafterweise so durchgeführt, daß man die Gesamtmenge des Polymerisationsansatzes (einschließlich gegebenenfalls des Molekulargewichtsreglers) abzüglich des Radikalbildners in das Polymerisationsgefäß vorlegt, auf die Polymerisationstemperatur erwärmt und anschließend (in der Regel auf einmal) Radikalbildner in das Polymerisationsgefäß einbringt und bis zu dem gewünschten Endumsatz, beispielweise 90% oder 98% polymerisiert.Anschließend kann die Emulsionpolymerisation durch Zusatz von Polymerisationsinhibitoren wie Diethylhydroxylamin gestoppt und nicht umgesetztes Monomer in bekannter Weise durch Desodorieren (vorzugsweise Strippen und/oder Wasserdampfdestillation) entfernt werden. Während der Polymerisation kann auch zur Erreichung des gewünschten Endumsatzes und zur zusätzlichen Stabilisation der Polymerdispersion weiterer Radikalbildner und/oder Dispergiermittel zugesetzt werden. Zur Stabilisierung des pH-Wertes können während der Polymerisation Puffer wie z.B. Alkaliphosphat zugesetzt werden. Ein Zusatz geringer Mengen starker Elektrolyte wie Kaliumsulfat, Kaliumchlorid und/oder Natriumsulfat erleichert in an sich bekannter Weise die Einstellung der gewünschten Teilchendurchmesser des Polymerisats. Im übrigen wird der Teilchendurchmesser des Polymerisats hauptsächlich durch die Menge des verwendeten Dispergiermittels bestimmt. In der Regel gehen mit zunehmender Dispergiermittelmenge abnehmende Teilchendurchmesser einher.

Im Anschluß an die Polymerisation kann der Feststoffgehalt des erhaltenen wässrigen heterogenen Polymerlatex durch Zugabe von Wasser oder Entzug von Wasser durch Destillation auf das gewünschte Niveau eingestellt werden. Die wässrigen Dispersionen können mit protischen Lösungsmitteln verdünnt werden. Als polare protische Lösemittel eignen sich beispielsweise Methanol, Ethanol, Isopropanol, Ethylenglycol, Propylenglycol, Glycerin, 1,3-Butylenglycol, 2,3-Butylenglycol, sowie Mischungen der genannten Alkohole. Im allgemeinen liegt das gewünschte Niveau des Polymerfeststoffgehalts bei etwa 20 bis 60 Gew-%, bezogen auf das Gesamtgewicht. Da die Emulsionspolymerisation ausschließlich in Wasser durchgeführt wird, ist diese Technik auf einen großen Maßstab übertragbar. In diesem Fall liegen die Monomerarten (A) und (B) und die gebildeten Polymerlatexpartikel im Wasser in sphärischer Form vor.

Die Herstellung der erfindungsgemäßen Komposit-Polymerlatices erfolgt durch bekannte, zweistufige Emulsionspolymerisation. Hierbei wird in einer ersten Stufe mit Hilfe der Emulsionspolymerisation eine Polymerdispersion erzeugt oder es wird ein bereits fertiges, beispielsweise im Handel erhältliches, unlösliches Polymer als Saatlatex in einem wässrigen Medium dispergiert. In der zweiten Stufe wird durch Zudosieren der üblichen Initiatoren sowie weiterer, radikalisch polymerisierbarer Monomere eine Hülle eines weiteren Polymeren in wässriger Emulsion aufgepfropft. Dabei ist mindestens eines der das Polymerisat der ersten Stufe (d.h. den Kern von Kern/Schale-Polymerpartikeln) oder das Polymerisat der zweiten Stufe (d.h. die Schale von Kern/Schale-Polymerpartikeln) bildenden Polymere aus mindestens einer Monomerart (A) aufgebaut ist, welche eine mit mindestens einer radikalisch polymerisierbaren Gruppe substituierte Verbindung ist, welche ausgewählt ist aus den oben näher erläuterten Kohlenhydraten oder Kohlenhydratderivaten, wobei die Monomerart eine oder mehrere Schutzgruppen enthalten kann. Die Menge an den Kern bildenden Polymeren und die Menge an die Schale bildenden Monomeren werden typischerweise so gewählt, daß die Schale 2 bis 95 Gew.%, vorzugsweise 5 bis 50 Gew.%, besonders bevorzugt 10 bis 30 Gew.% der Gesamtmenge der Kern/ Schale-Partikel ausmacht.

Vorzugsweise wird die Schale aus einem Homopolymer gemäß der oben angegebenen allgemeinen Formel (II) oder aus einem mit der Monomerart (B) copolymeriserten Polymer gemäß der oben angegebenen allgemeinen Formel (I) gebildet. Hierfür werden als ethylenisch ungesättigte Saccharidderivate der Monomerart (A) Verbindungen der oben angegebenen allgemeinen Formel (III) verwendet. Als Comonomere können die oben für die Monomerart (B) angegebenen ethylenisch ungesättigten Monomere eingesetzt werden.

Die zweistufige Emulsionspolymerisation zur Bildung der Komposit-Polymerlatices kann durch wasserlösliche oder durch öllösliche Starter gestartet werden. Bevorzugt sind öllösliche Starter, ausgewählt aus organischen Peroxiden, organischen Hydroperoxiden oder organischen Perestern. Geeignet sind beispielsweise Cumolhydroperoxid, Dicumolperoxid, tert.-Butylperneodekanoat, tert.-Butylperbenzoat oder Dioctanoyl- oder Dilauroylperoxid. Die Polymerisation der Schale erfolgt in Abhängigkeit des gewählten Initiatorsystems im Temperaturbereich von 30 bis 90°C. Gegebenenfalls können noch die oben angegebenen Emulgatoren zugegeben werden. Nach Beendigung der Monomerzugabe kann solange Initiator nachdosiert werden, bis der Restmonomergehalt bezogen auf das Gesamtgewicht des Latex unter 1 Gew.% liegt. Das Verfahrensprodukt kann direkt in Form des Latex eingesetzt und verwendet werden oder die Polymerisationsprodukte können beispielsweise durch Sprühtrocknung, Walzentrocknung oder durch Koagulation mit anschließender Trocknung aufgearbeitet und isoliert werden.

In einer bevorzugten Ausführungsform wird die zweistufige Emulsionspolymerisation zur Bildung der Komposit-Polymerlatices unter Verwendung von Pfropfungsmitteln durchgeführt, wobei die Pfropfungsmittel im Kern, in der Schale oder vorzugsweise in beiden enthalten sind. Geeignete Pfropfungsmittel sind beispielsweise Acryl- oder Methacrylsäureallylester, Acryl- oder Methacrylsäuremethallylester, Fumar-, Malein- oder Itaconsäuremono- oder -diallylester oder Fumar-, Malein- oder Itaconsäuremono- oder -dimethallylester.

Ein weiterer Gegenstand der vorliegenden Erfindung sind Polymerlatexhydrolysate. Hierbei werden in erster Stufe die Latexteilchen nach einem der obigen beschriebenen Verfahren hergestellt, und in zweiter Stufe werden die saccharidhaltigen Polymerlatices hydrolysiert. Dabei werden die chemisch gebundenen Saccharidbausteine entschützt. Falls ein in Wasser dispergierter Komposit-Polymerlatex mit einer Kern/Schale-Morphologie hydrolysiert wird, bei dem das Polymerisat der ersten Stufe (Kern) aus einem oder mehreren Polymeren aufgebaut ist, welche keine Monomerart (A) enthalten und das Polymerisat der zweiten Stufe (Schale) aus mindestens einem Homo- oder Copolymer gebildet wird, welches mindestens eine Monomerart (A) enthält, erhält man einen hydrophoben Kern und eine hydrophile Schale. Falls die Schale aus die Monomerart (A) enthaltendem Homopolymerisat aufgebaut ist, entsteht nach der säurekatalysierten Hydrolyse ein Latexpartikel mit einem hydrophoben Kern, der an der Partikeloberfläche mit wasserlöslichen Sachharideinheiten bedeckt ist.

Die erfindungsgemäßen Saccharidlatices weisen eine Reihe von Vorteilen gegenüber Lösungen von rein synthetischen Polymeren und von modifizierten natürlichen Polymeren auf, wie hoher Feststoffgehalt, Sicherheit, geringer Geruch, geringe Umweltverschmutzung, Lösungsmittelfreiheit der wässrigen Dispersionen.

Die erfindungsgemäßen Polymerlatices können verwendet werden zur Herstellung von Latexbindemitteln (z.B. wässrigen Bindemitteln im Textilbereich), Latexanstrichmitteln, Lacken, Farben, insbesondere Dispersionsfarben, Dispersionsklebstoffen, polymermodifiziertem Mörtel, Beton, Estrichmörtel und Putzen, Dichtmittel im Bau- und Wohnbereich, Klebemitteln in Putzen oder pharmazeutischen Produkten.

Die folgenden Beispiele sollen die Gegenstände der vorliegenden Erfindung näher erläutern, ohne daß die angefügten Ansprüche hierauf beschränkt sind.

### Beispiele

Die in dieser Anmeldung angegebenen Viskositäten sind dynamische Viskositäten und werden unter folgenden Bedingungen gemessen:
Messgerät: Rotationsviskosimeter RheoStress 100 der Firma Haake
Temperatur: 25°C
Schergefälle: 0,5 bis 1400 s⁻¹

### Beispiel 1: Herstellung eines Saccharidlatex

Gemäß dem nachstehend angegebenen Rezept bzw. Batch-Verfahren wurde ein Saccharidlatex hergestellt.

| | |
|---|---|
| Wasser | 460,00 g |
| Natriumdodecylsulfat (NDS) | 10,00 g |
| Methacroyldiacetonglucose (MDG) | 143,80 g |
| n-Butylacrylat (BA) | 56,20 g |
| Kaliumpersulfat | 1,05 g |

In einem mit Rückflußkühler und Rührer versehenen Doppelwandreaktor wurde unter Rühren mit 200 U/min und unter Argon oder Stickstoff Atmosphäre 460 ml entgastes, bidestilliertes Wasser in den Reaktor eingefüllt. Der Reaktor wurde auf 60°C erwärmt. 10,00 g NDS werden zugegeben und gelöst. Dann werden 143,80 g MDG und 56,20 g n-Butylacrylat (BA) zugegeben. Nach ca. 10 Minuten Temperierzeit wird 1,05 g Kaliumperoxodisulfat (wasserlöslicher Initiator) addiert. Der Reaktor wird verschlossen und unter Rühren die Reaktion gestartet. Nach 4 bzw. 6 Stunden wird die Reaktion dann 15 min lang auf 70°C gehalten und auf Raumtemperatur abgekühlt. Der pH des Dispersionslatex wurde durch Zugabe von 25%iger wässriger Ammoniaklösung auf 7 bis 8 eingestellt. Der erhaltene Saccharidlatex wies die folgenden physikalischen Eigenschaften auf: Feststoffgehalt: 29,7 %; Teilchengröße: 60-65 nm;
pH: 7,0; Viskosität: ca. 100-120 mPa.s.

### Beispiel 2: Herstellung eines Saccharidlatex nach dem Prä-emulsionsverfahren

| | |
|---|---|
| Wasser | 300,0 g |
| Natriumalkylphenolpolyglykolethersulfat, 35%ig (Rewopol® NOS10, WITCO GmbH) | 9,0 g |
| Kaliumpersulfat | 0,6 g |

| Monomermischung: | |
|---|---|
| Wasser | 300,0 g |
| Rewopol® NOS10 | 42,0 g |
| Methacroyldiacetonglucose (MDG) | 206,0 g |
| Butylacrylat (BA) | 400,0 g |
| Acrylsäure (AA) | 6,0 g |

| Initiatorlösung: | |
|---|---|
| Wasser | 150,0 g |
| Kaliumpersulfat | 2,1 g |

In einem mit Rückflußkühler, Tropftrichtern und Rührer versehenen 2 l Doppelwandreaktor wurde unter Rühren mit 200 U/min eine Anfangscharge von 300,0 g Wasser 9,00 g Rewopol NOS10 in den Reaktor eingefüllt. Der Reaktor wurde auf 60 °C erwärmt, dann wurde 0,60 g Kaliumpersulfat addiert. Die Monomermischung wurde kräftig vermischt und danach in das Reaktionsgefäß über eine Zeitspanne von 3 Stunden dosiert. Die Initiatorlösung wurde über eine Zeitspanne von 3 Stunden 30 min in den Reaktor dosiert. Die Reaktion wurde dann 45 min lang auf 70°C gehalten und auf Raumtemperatur abgekühlt. Der pH des Dispersionslatex wurde durch Zugabe von 25%iger wässriger Ammoniaklösung auf 7 bis 8 eingestellt. Der erhaltene Saccharidlatex wies die folgenden physikalischen Eigenschaften auf:
Feststoffgehalt: 44-45%; Teilchengröße: 100 nm;
pH: 8,0; Viskosität: ca. 100 mPa.s.

### Beispiel 3: Herstellung eines Saccharidlatex nach dem Shot-Growth-Verfahren

| | |
|---|---|
| Wasser | 390,00 g |
| Natriumdodecylsulfat (NDS) | 4,00 g |
| Butylacrylat | 112,00 g |
| Styrol | 48,00 g |
| Kaliumpersulfat | 0,76 g |

| Monomerzugabe: | |
|---|---|
| Methacroyldiacetonglucose (MDG) | 8,50 g |

In einem mit Rückflußkühler und Rührer versehenen Doppelwandreaktor wurde unter Rühren mit 200 U/min und unter Argon- oder Stickstoff-atmosphäre 390 ml entgastes, bidestilliertes Wasser in den Reaktor eingefüllt. Der Reaktor wurde auf 70 °C erwärmt. 4,00 g NDS werden zugegeben und gelöst. Dann wird eine Monomermischung aus 48,00 g Styrol und 112,00 g n-Butylacrylat zugegeben. Nach ca. 10 min Temperierzeit wird 0,46 g Kaliumperoxodisulfat addiert. Der Reaktor wird verschlossen und unter Rühren die Reaktion gestartet. Nach 2 Stunden Reaktionszeit (Umsatz ca. 80-90%) werden 8,50 g MDG auf einmal zugeführt. Nach 4 Stunden wird die Reaktion 20 min lang auf 80°C gehalten, dann auf Raumtemperatur abgekühlt. Der pH des Dispersionslatex wurde durch Zugabe von 25%iger wässriger Ammoniaklösung auf 7 bis 8 eingestellt. Der erhaltene Saccharidlatex wies die folgenden physikalischen Eigenschaften auf:
Feststoffgehalt: 29,8 %; Teilchengröße: 79 nm;
pH: 7,8; Viskosität: ca. 90 mPa.s.

### Beispiel 4: Herstellung von Saccharidlatex mit Kern-Schale-Polymerpartikeln im 2-stufigen Verfahren

| | | |
|---|---|---|
| Wasser | 400,00 g | |
| Natriumdodecylsulfat (NDS) | 8,40 g | |
| Kaliumpersulfat | 0,56 g | |

| Monomermischung: | Feed1 (Kern) | Feed2(Schale) |
|---|---|---|
| Wasser | 320,00 g | 160,00 g |
| Natriumdodecylsulfat (NDS) | 33,60 g | 1,12 g |
| Methylmethacrylat (MMA) | 205,85 g | - |
| Butylacrylat (BA) | 336,00 g | - |
| Methacroyldiacetonglucose (MDG) | - | 120,00 g |
| Methacrylsäure (MAA) | 2,25 g | - |

| Initiatorlösung: | Cofeed1 | Cofeed2 |
|---|---|---|
| Wasser | 100,00 g | 20,00 g |
| Kaliumpersulfat | 2,18 g | 0,61 g |

In einem mit Rückflußkühler, Tropftrichtern und Rührer versehener 2 1 Doppelwandreaktor wurde unter Rühren eine Anfangscharge von 400 g Wasser und 8,40 g NDS in den Reaktor eingefüllt. Der Reaktor wurde auf 60 °C unter Stickstoff erwärmt. Die Monomermischung Feed1 wurde kräftig vermischt und ein Teil davon (ca. 10 Gew.%) sowie im Anschluß hieran 0,56 g Kaliumpersulfat in das Reaktionsgefäß addiert. Das Reaktionsgemisch wurde 30 Minuten lang auf 60 °C gehalten. Der Rest von Feed1 wurde über eine Zeitspanne von 2 Stunden dosiert.

Gleichzeitig wurde die Initiatorlösung Cofeed 1 über eine Zeitspanne von 2 Stunden 30 min in den Reaktor dosiert. Die Reaktion wurde dann 20 min lang auf 70°C gehalten und auf 60 °C wiederabgekühlt. Dann wurde die Monomermischung Feed2 langsam über eine Zeitspanne von 90 Minuten dosiert, während die Initiatorlösung Cofeed 2 über eine Zeitspanne von 110 Minuten addiert wurde. Der pH des Dispersionslatex wurde durch Zugabe von 25%iger wässriger Ammoniaklösung auf 7 bis 8 eingestellt. Der erhaltene Saccharidlatex wies die folgenden physikalischen Eigenschaften auf:
Feststoffgehalt: 40 %; Teilchengröße: 144 nm; pH: 8,0; Viskosität: ca. 85 mPa.s.

### Beispiel 5: Herstellung von Kern-Schale Saccharidlatex mit multifunktionellen Verbindungen

Beispiel 4 wurde mit der Abänderung wiederholt, daß man zusätzlich 18,15 g der multifunktionellen Verbindung Allylmethacrylat (ALMA) in Feed 1 zur Bildung des Kerns einsetzte. In der Initiatorlösung wurde die Menge Kaliumpersulfats auf 2,29 g erhöht. Man erhielt einen Saccharidlatex, der folgende physikalische Eigenschaften aufweist:
Feststoffgehalt: 40 %; Teilchengröße: 225 nm; pH: 8,0; Viskosität: ca. 55-60 mPa.s.

### Beispiel 6: Herstellung eines Kern-Schale-Latex mit multifunktionellen Verbindungen und Reglern

Beispiel 5 wurde mit der Abänderung wiederholt, daß man zusätzlich 6,79 g n-Dodecylmercaptan als Regler in Feed 2 und die folgende Monomermischung einsetzte:

| Monomermischung: | Feed1 (Kern) | Feed2(Schale) |
|---|---|---|
| Wasser | 320,00 g | 160,00 g |
| Natriumdodecylsulfat (NDS) | 33,60 g | 1,12 g |
| Methylmethacrylat (MMA) | 249,88 g | - |
| Butylacrylat (BA) | 292,37 g | - |
| Methacroyldiacetonglucose (MDG) | - | 120,00 g |
| Methacrylsäure (MAA) | 2,25 g | - |
| Allylmethacrylat (ALMA) | 18,15 g | - |
| n-Dodecylmercaptan | - | 6,79 g |

Man erhielt einen Saccharidlatex, der folgende physikalische Eigenschaften aufweist:
Feststoffgehalt: 40 %; Teilchengröße: 225 nm; pH: 8,0; Viskosität: ca. 60-65 mPa.s.

## Patentansprüche

1. Polymerlatex, enthaltend mindestens ein in Wasser dispergiertes, durch Emulsionspolymerisation hergestelltes Polymer, welches aufgebaut ist aus
(A) mindestens einer ersten Monomerart, welche ein mit mindestens einer radikalisch polymerisierbaren Gruppe substituiertes Kohlehydrat oder Kohlehydratderivat ist, wobei die Monomerart eine oder mehrere Schutzgruppen enthalten kann, und
(B) mindestens einer zweiten, von (A) verschiedenen, mit (A) radikalisch copolymerisierbaren Monomerart,
wobei das Polymer die allgemeine Formel (I) aufweist
-[CH₂-C(Y)(D-A)]ₘ-[B]ₙ- (I)
wobei
Y für R oder COOR und R für H oder für einen C1- bis C18-Kohlenwasserstoffrest steht,
D für Sauerstoff, CONH oder COO steht,
A für ein Saccharid, ein Saccharidderivat oder für einen Zuckeralkohol steht und eine oder mehrere Schutzgruppen enthalten kann,
B für ein radikalisch polymerisierbares Monomer steht, welches keine Gruppe A enthält und
m und n für Zahlen größer Null stehen, die den jeweiligen Monomergehalt angeben,
unter der Maßgabe, dass D nicht für COO steht, wenn A von Galactose abgeleitet ist.

2. Polymerlatex nach Anspruch 1, **dadurch gekennzeichnet, daß** das Comonomer B ein Monomer oder eine Monomermischung ist, ausgewählt aus den Gruppen
(a) monoethylenisch ungesättigte C3- bis C12-Carbonsäuren oder deren Salze,
(b) monoethylenisch ungesättigte Ester von C3- bis C12-Carbonsäuren und C1- bis C14-Alkoholen,
(c) Acrylsäure- oder Methacrylsäuredialkylaminoalkylester mit insgesamt bis zu 30 C-Atomen im Dialkylaminoalkyl-Rest, welche in N-quaternisierter Form oder in Salzform vorliegen können,
(d) Amide der unter (a) genannten ungesättigten Carbonsäuren, z.B. Acrylsäureamid, Methacrylsäureamid, N,N-Dialkyl-acrylsäure- oder -methacrylsäureamid,
(e) fünf- bis achtgliedrige N-Vinyllactame, welche am Ring durch bis zu drei C1- bis C12-Alkylreste substituiert sein können,
(f) Maleinsäure-, Fumarsäure- und Itakonsäuredialkylester mit bis zu 12 C-Atomen pro Alkylrest,
(g) monoethylenisch ungesättigte C3- bis C12-Alkylvinylether,
(h) Vinylaromaten, wie z.B. Styrol, welches am aromatischen Ring durch ein bis drei C1- bis C12-Alkylreste substituiert sein kann,
(i) ein- oder mehrfach ungesättigte Kohlenwasserstoffe,
(j) Vinylhalogenide.

3. Polymerlatex nach einem der vorstehenden Ansprüche, **dadurch gekennzeichnet, daß** die Monomerart (A) bzw. der Rest A abgeleitet ist von Glucose, Fructose, Galactose, Sorbose oder Xylit und eine oder mehrere Schutzgruppen enthalten kann.

4. Polymerlatex nach einem der vorstehenden Ansprüche, **dadurch gekennzeichnet, daß** die Schutzgruppe ausgewählt ist aus Isopropyliden- oder Cyclohexyliden-Schutzgruppen.

5. Polymerlatex nach einem der der vorstehenden Ansprüche, **dadurch gekennzeichnet, daß** Y ausgewählt ist aus Wasserstoff, Methyl, -COOH, -COOCH₃ und -COOC₂H₅.

6. Polymerlatex nach einem der vorgenannten Ansprüche, **dadurch gekennzeichnet, daß** das Verhältnis von Monomerart (A) zu Monomerart (B) von 2:98 bis 98:2, vorzugsweise von 30:70 bis 70:30 beträgt.

7. Polymerlatex nach einem der vorgenannten Ansprüche, **dadurch gekennzeichnet, daß** die Monomerart (A) die allgemeine Formel (III) aufweist 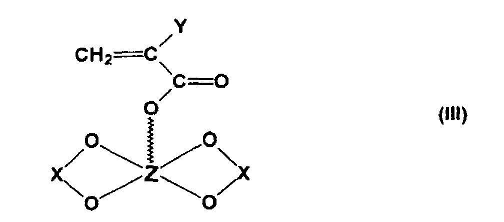 wobei Z einen Glucose- oder Fructoserest, X eine Isopropyliden- oder eine Cyclohexylidengruppe und Y Wasserstoff oder Methyl bedeuten.

8. Polymerlatex, enthaltend in Wasser dispergierte Komposit-Polymerpartikel, insbesondere Kern/Schale-Polymerpartikel, welche durch mindestens zweistufige Emulsionspolymerisation hergestellt sind und welche aufgebaut sind aus mindestens einem Polymer, welches seinerseits aufgebaut ist aus mindestens einer Monomerart (A), welche ein mit mindestens einer radikalisch polymerisierbaren Gruppe substituiertes Kohlenhydrat oder Kohlenhydratderivat ist,
wobei die Monomerart (A) eine oder mehrere Schutzgruppen enthalten kann.

9. Polymerlatex nach Anspruch 8, **dadurch gekennzeichnet, daß** das Polymerisat der zweiten Polymerisationsstufe (Schale) mindestens ein Polymer enthält, welches die Monomerart (A) enthält und das Polymerisat der ersten Polymerisationsstufe (Kern) aus einem beliebigen Polymer aufgebaut ist.

10. Polymerlatex nach Anspruch 9, **dadurch gekennzeichnet, daß** das Polymerisat der ersten Polymerisationsstufe (Kern) keine Bestandteile der Monomerart (A) enthält.

11. Polymerlatex nach Anspruch 8, **dadurch gekennzeichnet, daß** das Polymerisat der ersten Polymerisationsstufe (Kern) mindestens ein Polymer enthält, welches die Monomerart (A) enthält und das Polymerisat der zweiten Polymerisationsstufe (Schale) aus einem beliebigen Polymer aufgebaut ist.

12. Polymerlatex nach Anspruch 11, **dadurch gekennzeichnet, daß** das Polymerisat der zweiten Polymerisationsstufe (Schale) keine Bestandteile der Monomerart (A) enthält.

13. Polymerlatex nach einem der Ansprüche 8 bis 12, **dadurch gekennzeichnet, daß** das die Monomerart (A) enthaltende Polymer ein Homopolymer der allgemeinen Formel (II) ist
-[CH₂-C(Y)(D-A)]ₘ- (II)
wobei
Y für R oder COOR und R für H oder für einen C1- bis C18-Kohlenwasserstoffrest steht,
D für Sauerstoff, CONH oder COO steht,
A für ein Saccharid, ein Saccharidderivat oder für einen Zuckeralkohol steht und eine oder mehrere Schutzgruppen enthalten kann und
m für eine Zahl größer Null steht, die den Polymerisationsgrad angibt.

14. Polymerlatex nach einem der Ansprüche 8 bis 12, **dadurch gekennzeichnet, daß** das die Monomerart (A) enthaltende Polymer ein zusätzlich die Monomerart (B) enthaltendes Polymer gemäß einem der Ansprüche 1 bis 7 ist.

15. Polymerlatex nach einem der Ansprüche 8 bis 14, dadurch gekennzeinet, daß der Durchmesser der Komposit-Polymerpartikel 20 bis 3000 nm beträgt.

16. Polymerlatex nach einem der Ansprüche 8 bis 15, **dadurch gekennzeichnet, daß** der Gewichtsanteil des Polymerisats der zweiten Polymerisationsstufe (Schale) 2 bis 90% des Gesamtgewichts der Komposit-Polymerpartikel beträgt.

17. Polymerlatex nach einem der vorgenannten Ansprüche, **dadurch gekennzeichnet, daß** die Monomerart (A) frei von Schutzgruppen ist.

18. Verfahren zur Herstellung eines Polymerlatex gemäß einem der Ansprüche 1 bis 17, wobei die Monomere zur Bildung des dispers verteilten Polymerisats mittels Emulsionpolymerisation radikalisch polymerisiert werden.

19. Verfahren nach Anspruch 18, wobei die Monomerart (A) die allgemeine Formel (III) aufweist 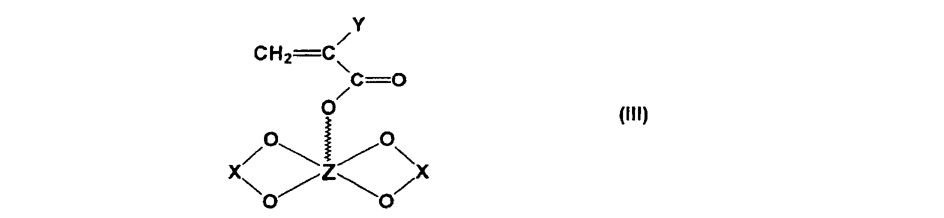 wobei
Y für R oder COOR und R für H oder für einen C1- bis C18-Kohlenwasserstoffrest steht,
Z für einen von einem Saccharid, einem Saccharidderivat oder einem Zuckeralkohol abgeleiteten Rest steht und
X für eine Schutzgruppe steht.

20. Verfahren nach Anspruch 19, **dadurch gekennzeichnet, daß** Z einen Glucose- oder Fructoserest, X eine Isopropyliden- oder eine Cyclohexylidengruppe und Y Wasserstoff oder Methyl bedeuten.

21. Verfahren nach einem der Ansprüche 18 bis 20, **dadurch gekennzeichnet, daß** die Monomerart (B) ein Monomer oder eine Monomermischung ist, die ausgewählt ist aus den Gruppen
(a) monoethylenisch ungesättigte C3- bis C12-Carbonsäuren oder deren Salze,
(b) monoethylenisch ungesättigte Ester von C3- bis C12-Carbonsäuren und C1- bis C14-Alkoholen,
(c) Acrylsäure- oder Methacrylsäuredialkylaminoalkylester mit insgesamt bis zu 30 C-Atomen im Dialkylaminoalkyl-Rest, welche in N-quaternisierter Form oder in Salzform vorliegen können,
(d) Amide der unter (a) genannten ungesättigten Carbonsäuren, z.B. Acrylsäureamid, Methacrylsäureamid, N,N-Dialkyl-acrylsäure- oder -methacrylsäureamid,
(e) fünf- bis achtgliedrige N-Vinyllactame, welche am Ring durch bis zu drei C1- bis C12-Alkylreste substituiert sein können,
(f) Maleinsäure-, Fumarsäure und Itakonsäuredialkylester mit bis zu 12 C-Atomen pro Alkylrest,
(g) monoethylenisch ungesättigte C3- bis C12-Alkylvinylether,
(h) Vinylaromaten, wie z.B. Styrol, welches am aromatischen Ring durch ein bis drei C1- bis C12-Alkylreste substituiert sein kann,
(i) ein- oder mehrfach ungesättigte Kohlenwasserstoffe,
(j) Vinylhalogenide.

22. Verfahren nach einem der Ansprüche 18 bis 21, **dadurch gekennzeichnet, daß** entweder
(a) das Verfahren als Batch-Verfahren ausgestaltet ist und die gesamte Monomerzugabe in einer einzelnen Charge erfolgt oder
(b) das Verfahren als Prä-Emulsionsverfahren ausgestaltet ist und die Monomerzugabe halbkontinuierlich, inkrementell erfolgt oder
(c) die Monomerzugabe kontinuierlich erfolgt oder
(d) das Verfahren zweistufig ist, wobei in der ersten Stufe eine Dispersion eines Saat-Polymerisats gebildet oder vorgelegt wird und in einer zweiten Stufe hieraus durch Emulsionspolymerisation ein Komposit-Polymerlatex gebildet wird oder
(e) das Verfahren als Shot-Growth-Verfahren ausgestaltet ist.

23. Verfahren zur Herstellung eines Komposit-Polymerlatex durch mindestens zweistufige Emulsionspolymerisation, **dadurch gekennzeichnet, daß** in einer ein geeignetes Saatlatex enthaltenden wässrigen Emulsion zur Bildung des Polymerisats der zweiten Stufe (Schale) mindestens ein radikalisch polymerisierbares Monomer polymerisiert wird, wobei mindestens eines der das Polymerisat der ersten Stufe (Kern) bzw. die Polymersaat oder das Polymerisat der zweiten Stufe (Schale) bildenden Polymere aus mindestens einer Monomerart (A) aufgebaut ist, welche eine mit mindestens einer radikalisch polymerisierbaren Gruppe substituierte Verbindung ist, welche ausgewählt ist aus Kohlenhydraten und Kohlenhydratderivaten, wobei die Monomerart eine oder mehrere Schutzgruppen enthalten kann.

24. Verfahren nach Anspruch 23, **dadurch gekennzeichnet, daß** mindestens eine von (A) verschiedene Monomerart (B) copolymerisiert wird.

25. Verfahren nach Anspruch 24, **dadurch gekennzeichnet, daß** die Monomerart (B) ein Monomer oder eine Monomermischung ist, die ausgewählt ist aus den Gruppen
(a) monoethylenisch ungesättigte C3- bis C12-Carbonsäuren oder deren Salze,
(b) monoethylenisch ungesättigte Ester von C3- bis C12-Carbonsäuren und C1- bis C14-Alkoholen,
(c) Acrylsäure- oder Methacrylsäuredialkylaminoalkylester mit insgesamt bis zu 30 C-Atomen im Dialkylaminoalkyl-Rest, welche in N-quaternisierter Form oder in Salzform vorliegen können,
(d) Amide der unter (a) genannten ungesättigten Carbonsäuren, z.B. Acrylsäureamid, Methacrylsäureamid, N,N-Dialkyl-acrylsäure- oder -methacrylsäureamid,
(e) fünf- bis achtgliedrige N-Vinyllactame, welche am Ring durch bis zu drei C1- bis C12-Alkylreste substituiert sein können,
(f) Maleinsäure-, Fumarsäure- und Itakonsäuredialkylester mit bis zu 12 C-Atomen pro Alkylrest,
(g) monoethylenisch ungesättigte C3- bis C12-Alkylvinylether,
(h) Vinylaromaten, wie z.B. Styrol, welches am aromatischen Ring durch ein bis drei C1- bis C12-Alkylreste substituiert sein kann,
(i) ein- oder mehrfach ungesättigte Kohlenwasserstoffe,
(j) Vinylhalogenide.

26. Verfahren nach einem der Ansprüche 18 bis 25, **dadurch gekennzeichnet, daß** das Verfahren unter Verwendung von wasserlöslichen oder öllöslichen radikalbildenden Startern durchgeführt wird.

27. Verfahren nach Anspruch 26, **dadurch gekennzeichnet, daß** die Starter in einer Menge von 0,01 bis 20 Mol% bezogen auf den Gesamtmonomergehalt eingesetzt werden und ausgewählt sind aus anorganischen und organischen Peroxiden, Persulfaten, Azoverbindungen oder Redoxsystemen.

28. Verfahren nach Anspruch 26 oder 27, **dadurch gekennzeichnet, daß** die öllöslichen Starter ausgewählt sind aus organischen Peroxiden, organischen Hydroperoxiden oder organischen Perestern.

29. Verfahren nach einem der Ansprüche 18 bis 28, **dadurch gekennzeichnet, daß** es bei Temperaturen von 20 bis 90°C durchgeführt wird.

30. Verfahren nach einem der Ansprüche 18 bis 29, **dadurch gekennzeichnet, daß** es unter Verwendung von 1 bis 20 Gew.% nichtionischer oder ionischer Tenside durchgeführt wird.

31. Verfahren nach einem der Ansprüche 18 bis 30, **dadurch gekennzeichnet, daß** es bezogen auf die Monomermenge unter Verwendung von 0,01 bis 10 Mol% Molekulargewichtsreglern durchgeführt wird.

32. Verfahren nach einem der Ansprüche 18 bis 31, **dadurch gekennzeichnet, daß** es unter Verwendung von 1 bis 20 Gew.%, bezogen auf den Monomergehalt, an Stabilisatoren, welche die Latices bei höherem Feststoffgehalt stabilisieren, durchgeführt wird.

33. Verfahren nach einem der Ansprüche 18 bis 32, **dadurch gekennzeichnet, daß** es unter Verwendung von 0,1 bis 20 Gew.% an quervernetzenden Verbindungen durchgeführt wird.

34. Verfahren nach Anspruch 33, **dadurch gekennzeichnet, daß** die quervernetzenden Verbindungen ausgewählt sind aus Divinylbenzol, Diacrylaten von Polyethylenglykol, Diacrylaten von Polypropylenglykol, N,N'-bis-methylenacrylamid, Divinylether der aliphatischen Diole, Diallylether, 1,7-Octadien, 1,9-Decadien, Triallylamin, Diallylphtalat oder Tetraallylethylendiamin.

35. Verfahren nach einem der Ansprüche 18 bis 34, **dadurch gekennzeichnet, daß** das Verfahren unter Verwendung von 0,1 bis 20 Gew.% Pfropfungsmitteln durchgeführt wird.

36. Verfahren nach Anspruch 35, **dadurch gekennzeichnet, daß** die Pfropfungsmittel ausgewählt sind aus Acryl- oder Methacrylsäureallylestern, Acryl- oder Methacrylsäuremethallylestern, Fumar-, Malein- oder Itaconsäuremono- oder -diallylestern und Fumar-, Malein- oder Itaconsäuremono- oder -dimethallylestern.

37. Verfahren nach einem der Ansprüche 18 bis 36, **dadurch gekennzeichnet, daß** die Schutzgruppen der Monomerart (A) entfernt werden.

38. Polymerlatex, hergestellt nach einem der Verfahren 18 bis 37.

39. Verwendung mindestens eines Polymerlatex gemäß einem der Ansprüche 1 bis 17 oder 38 zur Herstellung von Latexbindemitteln, Latexanstrichmitteln, Lacken, Farben, Klebstoffen, polymermodifiziertem Mörtel, Beton, Estrichmörtel und Putzen, Dichtmittel im Bau- und Wohnbereich oder von pharmazeutischen Produkten.

## Claims

1. Polymer latex comprising at least one water-dispersed polymer prepared by emulsion polymerization which is constructed from
(A) at least one first monomer type which is a carbohydrate or carbohydrate derivative substituted by at least one free-radically polymerizable group, where the monomer type can contain one or more protective groups, and
(B) at least one second monomer type which is different from (A) and can be free-radically copolymerized with (A),
where the polymer has the general formula (I)
-[CH₂-C(Y) (D-A)]ₘ-[B]ₙ- (I)
where
Y is R or COOR and R is H or a C1- to C18-hydrocarbon radical,
D is oxygen, CONH or COO,
A is a saccharide, a saccharide derivative or a sugar alcohol and can contain one or more protective groups,
B is a free-radically polymerizable monomer which does not contain a group A and
m and n are numbers greater than zero which indicate the respective monomer content,
with the proviso that D is not COO if A is derived from galactose.

2. Polymer latex according to Claim 1, **characterized in that** comonomer B is a monomer or a monomer mixture chosen from the groups
(a) monoethylenically unsaturated C3- to C12-carboxylic acids or salts thereof,
(b) monoethylenically unsaturated esters of C3- to C12-carboxylic acids and C1- to C14-alcohols,
(c) acrylic or methacrylic dialkylaminoalkyl esters with a total of up to 30 carbon atoms in the dialkylaminoalkyl radical, which can be in N-quaternized form or in salt form,
(d) amides of the unsaturated carboxylic acids specified under (a), e.g. acrylamide, methacrylamide, N,N-dialkylacrylamide or -methacrylamide,
(e) five- to eight-membered N-vinyllactams which can be substituted on the ring by up to three C1- to C12-alkyl radicals,
(f) maleic, fumaric and itaconic dialkyl esters having up to 12 carbon atoms per alkyl radical,
(g) monoethylenically unsaturated C3- to C12-alkyl vinyl ethers,
(h) vinylaromatics, such as, for example, styrene, which can be substituted on the aromatic rings by one to three C1- to C12-alkyl radicals,
(i) mono- or polyunsaturated hydrocarbons,
(j) vinyl halides.

3. Polymer latex according to one of the preceding claims, **characterized in that** the monomer type (A) or the radical A is derived from glucose, fructose, galactose, sorbose or xylitol and can contain one or more protective groups.

4. Polymer latex according to one of the preceding claims, **characterized in that** the protective group is chosen from isopropylidene or cyclohexylidene protective groups.

5. Polymer latex according to one of the preceding claims, **characterized in that** Y is chosen from hydrogen, methyl, -COOH, -COOCH₃ and -COOC₂H₅.

6. Polymer latex according to one of the above-mentioned claims, **characterized in that** the ratio of monomer type (A) to monomer type (B) is from 2:98 to 98:2, preferably from 30:70 to 70:30.

7. Polymer latex according to one of the above-mentioned claims, **characterized in that** monomer type (A) has the general formula (III) 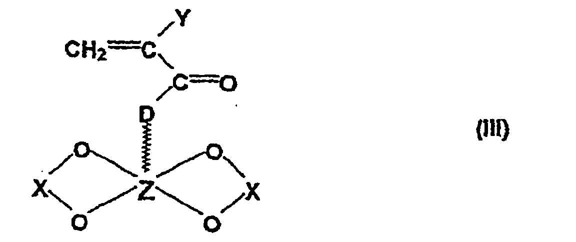 where Z is a glucose or fructose radical, X is an isopropylidene or a cyclohexylidene group and Y is hydrogen or methyl.

8. Polymer latex comprising water-dispersed composite polymer particles, in particular core-shell polymer particles which are prepared by at least two-stage emulsion polymerization and which are constructed from at least one polymer which in turn is constructed from at least one monomer type (A) which is a carbohydrate or carbohydrate derivative substituted by at least one free-radically polymerizable group, where the monomer type (A) can contain one or more protective groups.

9. Polymer latex according to Claim 8, **characterized in that** the polymer of the second polymerization stage (shell) comprises at least one polymer which comprises the monomer type (A), and the polymer of the first polymerization stage (core) is constructed from any polymer.

10. Polymer latex according to Claim 9, **characterized in that** the polymer of the first polymerization stage (core) does not comprise constituents of monomer type (A).

11. Polymer latex according to Claim 8, **characterized in that** the polymer of the first polymerization stage (core) comprises at least one polymer which comprises the monomer type (A), and the polymer of the second polymerization stage (shell) is constructed from any polymer.

12. Polymer latex according to Claim 11, **characterized in that** the polymer of the second polymerization stage (shell) does not comprise constituents of monomer type (A).

13. Polymer latex according to one of Claims 8 to 12, **characterized in that** the polymer comprising the monomer type (A) is a homopolymer of the general formula (II)
-[CH₂-C(Y) (D-A)]ₘ- (II)
where
Y is R or COOR and R is H or a C1- to C18-hydrocarbon radical,
D is oxygen, CONH or COO,
A is a saccharide, a saccharide derivative or a sugar alcohol and can contain one or more protective groups, and
m is a number greater than zero which indicates the degree of polymerization.

14. Polymer latex according to one of Claims 8 to 12, **characterized in that** the polymer comprising the monomer type (A) is a polymer additionally comprising the monomer type (B) according to one of Claims 1 to 7.

15. Polymer latex according to one of Claims 8 to 14, **characterized in that** the diameter of the composite polymer particles is 20 to 3000 nm.

16. Polymer latex according to one of Claims 8 to 15, **characterized in that** the proportion by weight of the polymer of the second polymerization stage (shell) is 2 to 90% of the total weight of the composite polymer particles.

17. Polymer latex according to one of the abovementioned claims, **characterized in that** the monomer type (A) is free from protective groups.

18. Process for the preparation of a polymer latex according to one of Claims 1 to 17, where the monomers for forming the dispersely distributed polymer are free-radically polymerized by means of emulsion polymerization.

19. Process according to Claim 18, where the monomer type (A) has the general formula (III) 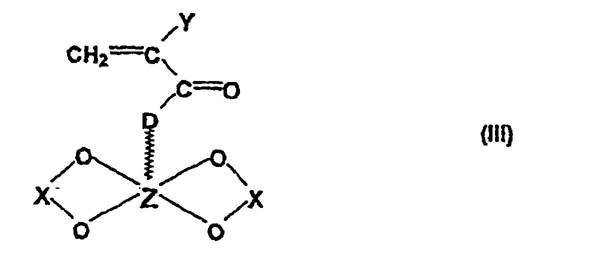 where
Y is R or COOR and R is H or a C1- to C18-hydrocarbon radical,
Z is a radical derived from a saccharide, a saccharide derivative or a sugar alcohol, and
x is a protective group.

20. Process according to Claim 19, **characterized in that** Z is a glucose or fructose radical, X is an isopropylidene or a cyclohexylidene group and Y is hydrogen or methyl.

21. Process according to one of Claims 18 to 20, **characterized in that** the monomer type (B) is a monomer or a monomer mixture which is chosen from the groups
(a) monoethylenically unsaturated C3- to C12-carboxylic acids or salts thereof,
(b) monoethylenically unsaturated esters of C3- to C12-carboxylic acids and C1- to C14-alcohols,
(c) acrylic or methacrylic dialkylaminoalkyl esters with a total of up to 30 carbon atoms in the dialkylaminoalkyl radical, which can be in N-quaternized form or in salt form,
(d) amides of the unsaturated carboxylic acids specified under (a), e.g. acrylamide, methacrylamide, N,N-dialkylacrylamide or -methacrylamide,
(e) five- to eight-membered N-vinyllactams which can be substituted on the ring by up to three C1- to C12-alkyl radicals,
(f) maleic, fumaric and itaconic dialkyl esters having up to 12 carbon atoms per alkyl radical,
(g) monoethylenically unsaturated C3- to C12-alkyl vinyl ethers,
(h) vinylaromatics, such as, for example, styrene, which can be substituted on the aromatic rings by one to three C1- to C12-alkyl radicals,
(i) mono- or polyunsaturated hydrocarbons,
(j) vinyl halides.

22. Process according to one of Claims 18 to 21, **characterized in that** either
(a) the process is configured as a batch process and the total monomer addition takes place in a single charge or
(b) the process is configured as a preemulsion process and the monomer addition takes place semicontinuously in increments or
(c) the monomer addition takes place continuously or
(d) the process is two-stage, where, in the first stage, a dispersion of a seed polymer is formed or initially introduced and, in a second stage, a composite polymer latex is formed therefrom from emulsion polymerization or
(e) the process is configured as a shot-growth process.

23. Process for the preparation of a composite polymer latex by at least two-stage emulsion polymerization, **characterized in that** at least one free-radically polymerizable monomer is polymerized in an aqueous emulsion, comprising a suitable seed latex, for the formation of the polymer of the second stage (shell), where at least one of the polymers forming the polymer of the first stage (core) or the polymer seed or the polymer of the second stage (shell) is constructed from at least one monomer type (A), which is a compound substituted by at least one free-radically polymerizable group which is chosen from carbohydrates and carbohydrate derivatives, where the monomer type can contain one or more protective groups.

24. Process according to Claim 23, **characterized in that** at least one monomer type (B) different from (A) is copolymerized.

25. Process according to Claim 24, **characterized in that** the monomer type (B) is a monomer or a monomer mixture which is chosen from the groups
(a) monoethylenically unsaturated C3- to C12-carboxylic acids or salts thereof,
(b) monoethylenically unsaturated esters of C3- to C12-carboxylic acids and C1- to C14-alcohols,
(c) acrylic or methacrylic dialkylaminoalkyl esters with a total of up to 30 carbon atoms in the dialkylaminoalkyl radical, which can be in N-quaternized form or in salt form,
(d) amides of the unsaturated carboxylic acids specified under (a), e.g. acrylamide, methacrylamide, N,N-dialkylacrylamide or -methacrylamide,
(e) five- to eight-membered N-vinyllactams which can be substituted on the ring by up to three C1- to C12-alkyl radicals,
(f) maleic, fumaric and itaconic dialkyl esters having up to 12 carbon atoms per alkyl radical,
(g) monoethylenically unsaturated C3- to C12-alkyl vinyl ethers,
(h) vinylaromatics, such as, for example, styrene, which can be substituted on the aromatic rings by one to three C1- to C12-alkyl radicals,
(i) mono- or polyunsaturated hydrocarbons,
(j) vinyl halides.

26. Process according to one of Claims 18 to 25, **characterized in that** the process is carried out using water-soluble or oil-soluble free-radical forming initiators.

27. Process according to Claim 26, **characterized in that** the initiators are used in an amount of from 0.01 to 20 mol%, based on the total monomer content, and are chosen from inorganic and organic peroxides, persulphates, azo compounds or redox systems.

28. Process according to Claim 26 or 27, **characterized in that** the oil-soluble initiators are chosen from organic peroxides, organic hydroperoxides or organic peresters.

29. Process according to one of Claims 18 to 28, **characterized in that** it is carried out at temperatures of from 20 to 90°C.

30. Process according to one of Claims 18 to 29, **characterized in that** it is carried out using from 1 to 20% by weight of nonionic or ionic surfactants.

31. Process according to one of Claims 18 to 30, **characterized in that** it is carried out using 0.01 to 10 mold of molecular weight regulators, based on the amount of monomer.

32. Process according to one of Claims 18 to 31, **characterized in that** it is carried out using from 1 to 20% by weight, based on the monomer content, of stabilizers which stabilize the lattices where the solids content is relatively high.

33. Process according to one of Claims 18 to 32, **characterized in that** it is carried out using from 0.1 to 20% by weight of crosslinking compounds.

34. Process according to Claim 33, **characterized in that** the crosslinking compounds are chosen from divinylbenzene, diacrylates of polyethylene glycol, diacrylates of polypropylene glycol, N,N'-bismethyleneacrylamide, divinyl ethers of aliphatic diols, diallyl ether, 1,7-octadiene, 1,9-decadiene, triallylamine, diallyl phthalate or tetraallylethylenediamine.

35. Process according to one of Claims 18 to 34, **characterized in that** the process is carried out using from 0.1 to 20% by weight of grafting agents.

36. Process according to Claim 35, **characterized in that** the grafting agents are chosen from acrylic or methacrylic allyl esters, acrylic or methacrylic methallyl esters, fumaric, maleic or itaconic mono- or diallyl esters and fumaric, maleic or itaconic mono- or dimethallyl esters.

37. Process according to one of Claims 18 to 36, **characterized in that** the protective groups of monomer type (A) are removed.

38. Polymer latex prepared by one of processes 18 to 37.

39. Use of at least one polymer latex according to one of Claims 1 to 17 or 38 for the preparation of latex binders, latex coatings, varnishes, paints, adhesives, polymer-modified mortars, concrete, screed mortar and plaster, sealants in the construction and housing sector or of pharmaceutical products.

## Revendications

1. Latex polymère, contenant au moins un polymère dispersé dans de l'eau, préparé par polymérisation en émulsion, et constitué de
(A) au moins un premier type de monomère, qui est un hydrate de carbone ou un dérivé d'hydrate de carbone substitué par au moins un groupe polymérisable par voie radicalaire, le type de monomère pouvant contenir un ou plusieurs groupes protecteurs, et
(B) au moins un deuxième type de monomère, différent de (A), copolymérisable avec (A) par voie radicalaire,
le polymère présentant la formule générale (I)
-[CH₂-C(Y) (D - A)]ₘ - [B]ₙ- (I)
dans laquelle
Y représente R ou COOR et R représente H ou un reste hydrocarboné en C₁ à C₁₈,
D représente de l'oxygène, CONH ou COO,
A représente un saccharide, un dérivé de saccharide ou un alcool de sucre et peut contenir un ou plusieurs groupes protecteurs,
B représente un monomère polymérisable par voie radicalaire, qui ne contient pas de groupe A, et
m et n représentent des nombres supérieurs à zéro, qui indiquent la teneur respective en monomère,
avec la condition que D ne représente pas COO lorsque A est dérivé de galactose.

2. Latex polymère selon la revendication 1, **caractérisé en ce que** le comonomère B est un monomère ou un mélange de monomères, choisi dans les groupes suivants:
(a) des acides carboxyliques monoéthyléniquement insaturés en C₃ à C₁₂ ou leurs sels,
(b) des esters monoéthyléniquement insaturés d'acides carboxyliques en C₃ à C₁₂ et d'alcools en C₁ à C₁₄,
(c) des dialkylaminoalkylesters d'acide acrylique ou méthacrylique ayant au total jusqu'à 30 atomes de carbone dans le radical dialkylaminoalkyle, et qui peuvent être présents sous forme N-quaternisée ou sous forme de sel,
(d) des amides des acides carboxyliques insaturés mentionnés en (a), par exemple de l'amide d'acide acrylique, de l'amide d'acide méthacrylique, de l'amide d'acide N,N-dialkylacrylique ou N,N-dialkylméthacrylique,
(e) des N-vinyllactames pentagonaux à octogonaux, qui peuvent être substitués sur le noyau par jusqu'à trois radicaux alkyle en C₁ à C₁₂,
(f) des dialkylesters d'acide maléique, d'acide fumarique et d'acide itaconique ayant jusqu'à 12 atomes de C dans le radical alkyle,
(g) des alkylvinyléthers en C₃ à C₁₂ monoéthyléniquement insaturés,
(h) des vinylaromatiques, comme par exemple le styrène, lequel peut être substitué sur le noyau aromatique par un à trois radicaux alkyle en C₁ à C₁₂,
(i) des hydrocarbures mono- ou polyinsaturés,
(j) des halogénures de vinyle.

3. Latex polymère selon l'une quelconque des revendications qui précèdent, **caractérisé en ce que** le type de monomère (A) ou le reste A est dérivé de glucose, de fructose, de galactose, de sorbose ou de xylite et peut contenir un ou plusieurs groupes protecteurs.

4. Latex polymère selon l'une quelconque des revendications qui précèdent, **caractérisé en ce que** le groupe protecteur est choisi parmi des groupes protecteurs d'isopropylidène ou de cyclohexylidène.

5. Latex polymère selon l'une quelconque des revendications qui précèdent, **caractérisé en ce que** Y est choisi parmi de l'hydrogène, un radical méthyle, -COOR, -COOCH₃ et -COOC₂H₅.

6. Latex polymère selon l'une quelconque des revendications qui précèdent, **caractérisé en ce que** la proportion du type de monomère (A) au type de monomère (B) est de 2 : 98 à 98 : 2, de préférence de 30 : 70 à 70 : 30.

7. Latex polymère selon l'une quelconque des revendications qui précèdent, **caractérisé en ce que** le type de monomère (A) présente la formule générale (III) 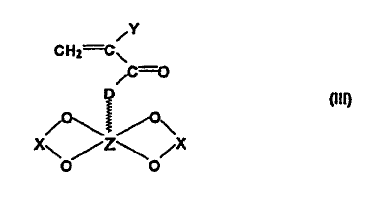 dans laquelle Z représente un reste fructose ou glucose, X un groupe isopropylidène ou cyclohexylidène et Y de l'hydrogène ou un radical méthyle.

8. Latex polymère, contenant des particules polymères composites dispersées dans de l'eau, en particulier des particules polymères à noyau/gaine, qui sont préparées par polymérisation en émulsion en au moins deux étapes, et qui sont constituées d'au moins un polymère, qui de son côté est constitué d'au moins un type de monomère (A), qui est un hydrate de carbone ou un dérivé d'hydrate de carbone substitué par au moins un groupe polymérisable par voie radicalaire, le type de monomère (A) pouvant contenir un ou plusieurs groupes protecteurs.

9. Latex polymère selon la revendication 8, **caractérisé en ce que** le polymère de la deuxième étape de polymérisation (gaine) contient au moins un polymère, qui contient le type de monomère (A) et **en ce que** le polymère de la première étape de polymérisation (noyau) est constitué d'un polymère quelconque.

10. Latex polymère selon la revendication 9, **caractérisé en ce que** le polymère de la première étape de polymérisation (noyau) ne contient pas de constituants du type de monomère (A).

11. Latex polymère selon la revendication 8, **caractérisé en ce que** le polymère de la première étape de polymérisation (noyau) contient au moins un polymère qui contient le type de monomère (A) et **en ce que** le polymère de la deuxième étape de polymérisation (gaine) est constitué d'un polymère quelconque.

12. Latex polymère selon la revendication 11, **caractérisé en ce que** le polymère de la deuxième étape de polymérisation (gaine) ne contient pas de constituants du type de monomère (A).

13. Latex polymère selon l'une quelconque des revendications 8 à 12, **caractérisé en ce que** le polymère contenant le type de monomère (A) est un homopolymère de la formule générale (II)
-[CH₂-C(Y)(D-A)]ₘ- (II)
dans laquelle
Y représente R ou COOR et R représente H ou un reste hydrocarboné en C₁ à C₁₈,
D représente de l'oxygène, CONH ou COO,
a représente un saccharide, un dérivé de saccharide ou un alcool de sucre et peut contenir un ou plusieurs groupes protecteurs, et
m est un nombre supérieur à zéro, qui indique le degré de polymérisation.

14. Latex polymère selon l'une quelconque des revendications 8 à 12, **caractérisé en ce que** le polymère contenant le type de monomère (A) est un polymère contenant en outre le type de monomère (B) selon l'une quelconque des revendications 1 à 7.

15. Latex polymère selon l'une quelconque des revendications 8 à 14, **caractérisé en ce que** le diamètre des particules polymères composites est de 20 à 3000 nm.

16. Latex polymère selon l'une quelconque des revendications 8 à 15, **caractérisé en ce que** la fraction pondérale du polymère de la deuxième étape de polymérisation (gaine) est de 2 à 90% du poids total des particules polymères composites.

17. Latex polymère selon l'une quelconque des revendications qui précèdent, **caractérisé en ce que** le type de monomère (A) est exempt de groupes protecteurs.

18. Procédé de préparation d'un latex polymère selon l'une quelconque des revendications 1 à 17, dans lequel les monomères sont polymérisés par voie radicalaire pour la formation du polymère à distribution disperse par polymérisation en émulsion.

19. Procédé selon la revendication 18, dans lequel le type de monomère (A) présente la formule générale (III) 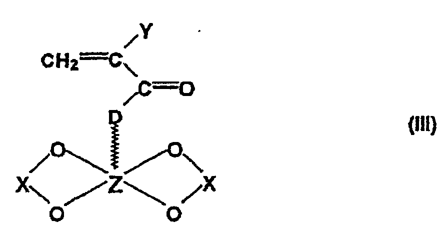 dans laquelle
Y représente R ou COOR et R représente H ou un reste hydrocarboné en C₁ à C₁₈,
Z représente un reste dérivé d'un saccharide, d'un dérivé de saccharide ou d'un alcool de sucre et
x représente un groupe protecteur.

20. Procédé selon la revendication 19, **caractérisé en ce que** Z représente un reste glucose ou fructose, X un groupe isopropylidène ou cyclohexylidène, et Y de l'hydrogène ou un radical méthyle.

21. Procédé selon l'une quelconque des revendications 18 à 20, **caractérisé en ce que** le type de monomère (B) est un monomère ou un mélange de monomères choisi dans les groupes suivants:
(a) des acides carboxyliques monoéthyléniquement insaturés en C₃ à C₁₂ ou leurs sels,
(b) des esters monoéthyléniquement insaturés d'acides carboxyliques en C₃ à C₁₂ et d'alcools en C₁ à C₁₄,
(c) des dialkylaminoalkylesters d'acide acrylique ou méthacrylique ayant au total jusqu'à 30 atomes de carbone dans le radical dialkylaminoalkyle, et qui peuvent être présents sous forme N-quaternisée ou sous forme de sel,
(d) des amides des acides carboxyliques insaturés mentionnés en (a), par exemple de l'amide d'acide acrylique, de l'amide d'acide méthacrylique, de l'amide d'acide N,N-dialkylacrylique ou N,N-dialkylméthacrylique,
(e) des N-vinyllactames pentagonaux à octogonaux, qui peuvent être substitués sur le noyau par jusqu'à trois radicaux alkyle en C₁ à C₁₂,
(f) des dialkylesters d'acide maléique, d'acide fumarique et d'acide itaconique ayant jusqu'à 12 atomes de C dans le radical alkyle,
(g) des alkylvinyléthers en C₃ à C₁₂ monoéthyléniquement insaturés,
(h) des vinylaromatiques, comme par exemple le styrène, lequel peut être substitué sur le noyau aromatique par un à trois radicaux alkyle en C₁ à C₁₂,
(i) des hydrocarbures mono- ou polyinsaturés,
(j) des halogénures de vinyle.

22. Procédé selon l'une quelconque des revendications 18 à 21, **caractérisé en ce que**:
(a) soit le procédé est conçu en tant que procédé discontinu et l'on ajoute la totalité des monomères en une seule charge,
(b) soit le procédé est conçu en tant que procédé en pré-émulsion et l'addition des monomères est entreprise de manière semi-continue et incrémentale,
(c) soit l'addition des monomères est entreprise en continu,
(d) soit le procédé est conçu en deux étapes, auquel cas, dans la première étape, une dispersion d'un polymère de semence est formée ou chargée et, dans une deuxième étape, on forme à partir d'elle, par polymérisation en émulsion, un latex polymère composite,
(e) soit le procédé est conçu en tant que procédé "shot-growth".

23. Procédé de préparation d'un latex polymère composite par polymérisation en émulsion en au moins deux étapes, **caractérisé en ce que** l'on polymérise, dans une émulsion aqueuse contenant un latex de semence approprié pour la formation du polymère de la deuxième étape (gaine), au moins un monomère polymérisable par voie radicalaire, au moins l'un des polymères formant le polymère de la première étape (noyau) ou la semence polymère ou le polymère de la deuxième étape (gaine) étant constitué d'au moins un type de monomère (A), qui est un composé substitué par au moins un groupe polymérisable par voie radicalaire, choisi parmi des hydrates de carbone et des dérivés d'hydrates de carbone, le type de monomère pouvant contenir un ou plusieurs groupes protecteurs.

24. Procédé selon la revendication 23, **caractérisé en ce que** l'on copolymérise au moins un type de monomère (B) différent de (A).

25. Procédé selon la revendication 24, **caractérisé en ce que** le type de monomère (B) est un monomère ou un mélange de monomères choisi dans les groupes suivants:
(a) des acides carboxyliques monoéthyléniquement insaturés en C₃ à C₁₂ ou leurs sels,
(b) des esters monoéthyléniquement insaturés d'acides carboxyliques en C₃ à C₁₂ et d'alcools en C₁ à C₁₄,
(c) des dialkylaminoalkylesters d'acide acrylique ou méthacrylique ayant au total jusqu'à 30 atomes de carbone dans le radical dialkylaminoalkyle, et qui peuvent être présents sous forme N-quaternisée ou sous forme de sel,
(d) des amides des acides carboxyliques insaturés mentionnés en (a), par exemple de l'amide d'acide acrylique, de l'amide d'acide méthacrylique, de l'amide d'acide N,N-dialkylacrylique ou N,N-dialkylméthacrylique,
(e) des N-vinyllactames pentagonaux à octogonaux, qui peuvent être substitués sur le noyau par jusqu'à trois radicaux alkyle en C₁ à C₁₂,
(f) des dialkylesters d'acide maléique, d'acide fumarique et d'acide itaconique ayant jusqu'à 12 atomes de C dans le radical alkyle,
(g) des alkylvinyléthers en C₃ à C₁₂ monoéthyléniquement insaturés,
(h) des vinylaromatiques, comme par exemple le styrène, lequel peut être substitué sur le noyau aromatique par un à trois radicaux alkyle en C₁ à C₁₂,
(i) des hydrocarbures mono- ou polyinsaturés,
(j) des halogénures de vinyle.

26. Procédé selon l'une quelconque des revendications 18 à 25, **caractérisé en ce que** le procédé est entrepris avec utilisation de molécules amorces hydrosolubles ou oléosolubles formant des radicaux.

27. Procédé selon la revendication 26, **caractérisé en ce que** les molécules amorces sont mises en oeuvre en une quantité de 0,01 à 20% molaires, par rapport à la teneur totale en monomères, et sont choisies parmi des peroxydes, persulfates, composés azoïques ou systèmes rédox inorganiques et organiques.

28. Procédé selon la revendication 26 ou 27, **caractérisé en ce que** les molécules amorces oléosolubles sont choisies parmi des peroxydes organiques, des hydroperoxydes organiques ou des peresters organiques.

29. Procédé selon l'une quelconque des revendications 18 à 28, **caractérisé en ce qu'**il est entrepris à des températures de 20 à 90°C.

30. Procédé selon l'une quelconque des revendications 18 à 29, **caractérisé en ce qu'**il est entrepris avec utilisation de 1 à 20% en poids d'agents tensioactifs non ioniques ou ioniques.

31. Procédé selon l'une quelconque des revendications 18 à 30, **caractérisé en ce qu'**il est entrepris avec utilisation de 0,01 à 10% molaires, par rapport à la quantité de monomères, de régulateurs du poids moléculaire.

32. Procédé selon l'une quelconque des revendications 18 à 31, **caractérisé en ce qu'**il est entrepris avec utilisation 1 à 20% en poids, par rapport à la teneur en monomères, de stabilisants, qui stabilisent les latex à haute teneur en solides.

33. Procédé selon l'une quelconque des revendications 18 à 32, **caractérisé en ce qu'**il est entrepris avec utilisation de 0,1 à 20% en poids de composés à réticulation transversale.

34. Procédé selon la revendication 33, **caractérisé en ce que** les composés à réticulation transversale sont choisis parmi le divinylbenzène, des diacrylates de polyéthylène glycol, des diacrylates de polypropylène glycol, l'acrylamide de N,N'-bis-méthylène, des divinyléthers des diols aliphatiques, des éthers diallyliques, le 1,7-octadiène, le 1,9-décadiène, la triallylamine, le phtalate de diallyle ou la tétraallyléthylènediamine.

35. Procédé selon l'une quelconque des revendications 18 à 34, **caractérisé en ce que** le procédé est entrepris avec utilisation de 0,1 à 20% en poids d'agents de greffage.

36. Procédé selon la revendication 35, **caractérisé en ce que** les agents de greffage sont choisis parmi des allylesters d'acide acrylique ou méthacrylique, des méthallyesters d'acide acrylique ou méthacrylique, des mono- ou diallylesters d'acide fumarique, maléique ou itaconique et des mono- ou diméthallylesters d'acide fumarique, maléique ou itaconique.

37. Procédé selon l'une quelconque des revendications 18 à 36, **caractérisé en ce que** les groupes protecteurs du type de monomère (A) sont éliminés.

38. Latex polymère préparé par l'un des procédés 18 à 37.

39. Utilisation d'au moins un latex polymère selon l'une quelconque des revendications 1 à 17 ou 38 pour la préparation de liants de latex, de produits de revêtement au latex, de laques, peintures, adhésifs, mortiers, bétons, mortiers d'enduisage et crépis modifiés par des polymères, masses d'étanchéité pour construction et habitations, ou produits pharmaceutiques.
